(19) Europäisches Patentamt
European Patent Office
Office européen des brevets

(11) EP 1 772 136 A1

(12) # DEMANDE DE BREVET EUROPEEN

(43) Date de publication:
**11.04.2007 Bulletin 2007/15**

(51) Int Cl.:
***A61K 8/04*** (2006.01) ***A61K 8/81*** (2006.01)
***A61Q 5/02*** (2006.01) ***A61Q 5/06*** (2006.01)

(21) Numéro de dépôt: **06121666.9**

(22) Date de dépôt: **03.10.2006**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **06.10.2005 FR 0553040**

(71) Demandeur: **L'ORÉAL**
**75008 Paris (FR)**

(72) Inventeurs:
- **Giroud, Franck**
  **92110 Clichy (FR)**
- **Paul, Laurence**
  **95320 Saint Leu la Forêt (FR)**
- **Mougin, Nathalie**
  **75011 Paris (FR)**
- **Jegou, Gwenaëlle**
  **93190 Livry Gargan (FR)**

(74) Mandataire: **Le Blainvaux Bellegarde, Françoise**
**L'OREAL - D.I.P.I.**
**25-29 Quai Aulagnier**
**92600 Asnières (FR)**

(54) **Compositions cosmétiques contenant au moins un tensioactif et au moins une dispersion non aqueuse de polymère**

(57) L'invention a pour objet des compositions cosmétiques selon comprenant :
A) au moins un tensioactif anionique, non ionique et/ou amphotère et
B) au moins une dispersion non aqueuse d'un polymère éthylénique stabilisé en surface par un agent stabilisant, ledit polymère présentant une température de transition vitreuse inférieure ou égale à -20°C.

Les cheveux traités se démêlent facilement lors rinçage, et présentent de la douceur; après séchage. Les compositions selon l'invention permettent également, une fois la chevelure séchée, une mise en forme et une tenue des cheveux particulièrement intéressante.

EP 1 772 136 A1

## Description

La présente invention a trait à des compositions comprenant au moins un tensioactif et au moins une dispersion non aqueuse de polymère.

Il est connu d'employer des polymères dans le domaine cosmétique, et notamment en capillaire dans les produits non rincés, par exemple pour apporter de la tenue ou du coiffant à la chevelure.

Dans le domaine des compositions capillaires dites "rincées", telles que les shampooings ou après-shampooings, on utilise aussi des polymères cationiques synthétiques, solubles dans l'eau, qui sont connus pour apporter une bonne cosmétique aux cheveux; toutefois, ces polymères n'apportent aucun effet de mise en forme des cheveux. Il en est de même avec les polymères dérivés naturels cationiques tels les guars modifiées qui apportent également un caractère cosmétique sans permettre une mise en forme. Dans le domaine des compositions rincées, les polymères n'apportent pas suffisamment de coiffant associée à une cosmétique acceptable.

La présente invention a pour but de proposer des compositions cosmétiques comprenant des polymères capables d'apporter un réel effet coiffant tout en conservant une cosmétique acceptable aux compositions.

Après de nombreuses recherches, la demanderesse a mis en évidence que l'utilisation de dispersions non aqueuses de polymères particuliers tels que définis ci-après, pouvait permettre la réalisation de compositions coiffantes rincées ou non rincées ayant des propriétés cosmétiques adéquates.

On connaît des compositions d'après-shampooings possédant des propriétés coiffantes comprenant des polymères solubilisés dans des solvants insolubles dans l'eau. De telles compositions sont décrites dans les demandes de brevet W09115185, W09115186, W09707782.
Ces compositions présentent des propriétés cosmétiques médiocres notamment le toucher est gras et collant.

La présente invention a pour but de proposer des compositions cosmétiques comprenant des polymères capables d'apporter un réel effet coiffant tout en conservant une cosmétique acceptable aux compositions.

Après de nombreuses recherches, la demanderesse a mis en évidence que l'utilisation de dispersion de polymères particuliers telles que définies ci-après, pouvait permettre la réalisation de compositions coiffantes rincées ou non rincées ayant une cosmétique adéquate.

Des dispersions non aqueuses de polymères sont décrites dans l'art antérieur. Cependant, ces dispersions ne permettent pas d'obtenir un effet coiffant.

De manière surprenante, les polymères selon l'invention possèdent des propriétés cosmétiques intéressantes, par exemple lors de l'application dans une formulation de type shampooing; on a en effet constaté que les cheveux se démêlent facilement lors du shampooing, et qu'ils présentent de la douceur; après séchage, les compositions selon l'invention permettent également, une fois la chevelure séchée, une mise en forme et une tenue des cheveux particulièrement intéressante.

Les compositions cosmétiques selon l'invention sont caractérisées par le fait qu'elles comprennent :

I) au moins un tensioactif anionique, amphotère, non ionique et leurs mélanges et

II) au moins une dispersion de particules d'au moins un polymère éthylénique stabilisé en surface par un agent stabilisant, dans un milieu non aqueux constitué d'au moins un composé non aqueux, liquide à 25°C, ayant un paramètre de solubilité global selon l'espace de solubilité de HANSEN inférieur ou égal à 20 $(MPa)^{1/2}$, ou un mélange de tels composés;
ledit polymère éthylénique présente une température de transition vitreuse (Tg) inférieure ou égale à -20°C

Les dispersions selon l'invention sont donc constituées de particules, généralement sphériques, d'au moins un polymère éthylénique, stabilisé en surface par un stabilisant, dans un milieu non aqueux.

Les dispersions selon l'invention peuvent notamment se présenter sous forme de nanoparticules de polymères en dispersion stable dans un milieu non aqueux. Les nanoparticules sont de préférence d'une taille moyenne en nombre comprise entre 5 et 600 nm, notamment 10 à 500 nm, encore mieux 15 à 450 nm, étant donné qu'au-delà d'environ 600 nm, les dispersions de particules deviennent beaucoup moins stables.
Notamment, ces particules restent à l'état de particules élémentaires, sans former d'agglomérats, lorsqu'elles sont en dispersion dans lesdits milieux non aqueux.

Par polymère "éthylénique", on entend un polymère obtenu par polymérisation d'au moins 2 monomères, identiques ou différents, comprenant une insaturation éthylénique.
Ledit polymère éthylénique peut être choisi par l'homme du métier en fonction de ses propriétés, selon l'application ultérieure souhaitée pour la composition. Ces polymères peuvent être en particulier réticulés.

Toutefois, ledit polymère éthylénique présente au moins une température de transition vitreuse (Tg) inférieure ou égale à -20°C, de préférence comprise entre - 150°C et -20 °C, notamment entre -100°C et -25 °C, préférentiellement entre -95 °C et -30°C, voire entre -80°C et -35°C, et encore mieux entre -70°C et -40°C.

De préférence, le polymère ne présente qu'une température de transition vitreuse.
Toutefois, il peut présenter plusieurs températures de transition vitreuse, notamment deux Tg; dans ce cas, la Tg la plus basse doit être inférieure à -20°C.

Dans la présente invention, les Tg (ou température de transition vitreuse) indiquées sont des Tg théoriques déterminées à partir des Tg théoriques des monomères constitutifs du polymère, que l'on peut trouver dans un manuel de référence, tel que le Polymer Handbook, 3ème ed, 1989, John Wiley, selon la relation suivante, dite Loi de Fox :

$$\frac{1}{Tg} = \sum_i \left(\frac{\varpi i}{Tgi}\right)$$

wi étant la fraction massique du monomère i dans le polymère et Tgi étant la température de transition vitreuse de l'homopolymère du monomère i, exprimée en Kelvin (K).

Dans la présente description, on désigne par "monomère de Tg", le monomère dont l'homopolymère a une telle température de transition vitreuse.

Les polymères selon l'invention peuvent être des homopolymères ou des copolymères linéaires, branchés, ou même en étoiles. Ils peuvent être statistiques ou alternés. De préférence, ce sont des copolymères statistiques linéaires.

D'une manière générale, les monomères de Tg inférieure ou égale à -20°C, peuvent représenter 50 à 100% en poids du poids total des monomères initiaux.

Dans un premier mode de réalisation de l'invention, les polymères présents dans la dispersion peuvent être issus de la polymérisation d'un ou plusieurs monomères de Tg inférieure ou égale à -20°C, de préférence comprise entre -150°C et -20°C, notamment entre -100°C et -25 °C, préférentiellement entre -95 °C et -30 °C, voire entre -80°C et -40°C, entre - 60 °Cet - 40°C, et encore -70°C et -45°C.
Dans ce mode de réalisation, le monomère de Tg inférieure ou égale à -20°C, ou leur mélange, représente 100% en poids du poids total de monomères initiaux.

Dans un second mode de réalisation de l'invention, les polymères présents dans la dispersion peuvent être issus de la polymérisation d'un ou plusieurs monomères de Tg inférieure ou égale à -20°C, et d'un ou plusieurs monomères dits additionnels de Tg supérieure à -20°C, mais présents en une quantité telle que la Tg globale du polymère est inférieure ou égale à -20°C, de préférence comprise entre - 150°C et -20°C, notamment entre -100°C et -25 °C, préférentiellement entre -50 °C et -30°C.
Par exemple, on peut combiner dans le polymère final, un monomère de Tg de l'ordre de 100°C, qui peut être présent à raison de 10-20% en poids du poids total de monomères, et un monomère de Tg de l'ordre de -50°C, qui peut être présent à raison de 80-90% en poids, de manière à obtenir un polymère ayant une Tg d'environ -40°C à -30°C.
Dans ce mode de réalisation, de préférence le monomère additionnel, ou le mélange de tels monomères, peut être présent à raison de 0,01 à 50% en poids, par rapport au poids total des monomères, notamment de 0,1 à 40% en poids, voire de 1 à 30% en poids, ou encore de 5 à 15% en poids. Le monomère de Tg inférieure ou égale à -20°C, ou le mélange de tels monomères, peut alors être présent à raison de 50 à 99,99% en poids, notamment de 60 à 99,9% en poids, voire de 70 à 99% en poids, ou encore de 85 à 95% en poids, par rapport au poids total de monomères.
L'homme du métier saura, sur la base de la loi de Fox et de ses connaissances générales, déterminer les quantités maximales de monomère additionnel susceptible d'être présent dans le polymère de la dispersion, de manière à toujours obtenir au final une dispersion de polymère ayant une Tg inférieure ou égale à -20°C.

Par ailleurs, on a constaté que lorsque la dispersion de polymère selon l'invention comprend un ou plusieurs monomères dits hydrophobes tels que définis ci-après, elle présente de bonnes propriétés de rémanence du dépôt, ainsi que de douceur, et une bonne compatibilité avec les compositions de type après-shampooing.

Ainsi, de préférence, le polymère éthylénique selon l'invention comprend 40% à 100% en poids, par rapport au poids total de monomères, notamment de 60 à 99% en poids, voire de 70 à 98% poids, et encore mieux de 60 à 95% en poids de monomère hydrophobe, seul ou en mélange.
Ce ou ces monomères hydrophobes pourront être choisis parmi les monomères ayant une Tg inférieure ou égale à -20°C et/ou parmi les monomères ayant une Tg supérieure à -20°C.
De préférence, le ou les monomères hydrophobes ont une Tg inférieure à -20°C.

Par "monomère hydrophobe", on entend au sens de la présente invention un monomère ayant une valeur du logarithme du coefficient de partage apparent octanol-1/eau, aussi appelé log P, supérieure ou égale à 2, par exemple comprise entre 2 et 11, de préférence comprise entre 2,5 et 10, notamment entre 3 et 8, voire entre 3,5 et 5.

Les valeurs de log P sont connues et sont déterminées selon un test standard qui détermine la concentration du monomère dans l'octanol-1 et l'eau.
Les valeurs peuvent également être calculées à l'aide du logiciel ACD (Advanced Chemistry Development) Software solaris V4.67; elles peuvent également être obtenues à partir de Exploring QSAR : hydrophobic, electronic and steric

constants (ACS professional référence book, 1995). Il existe encore un site Internet qui fournit des valeurs estimées (adresse : http://esc.syrres.com/interkow/kowdemo.htm).

Nous indiquons ci-après la valeur du log P de certains monomères usuels, déterminée à l'aide du logiciel ACD :

| | acrylate (* acrylamide) | méthacrylate (* méthacrylamide) |
|---|---|---|
| méthyl (méth)acrylate | 0.793 +/- 0.223 | 1.346 +/- 0.250 |
| éthyl (méth)acrylate | 1.325 +/-0.223 | 1.877 +/-0.250 |
| propyl (méth)acrylate | 1.856 +/- 0.223 | 2.408 +/- 0.250 |
| isopropyl (méth)acrylate | 1.672 +/- 0.228 | 2.224 +/- 0.254 |
| n-butyl (méth)acrylate | 2.387 +/- 0.223 | 2.940 +/- 0.250 |
| isobutyl (méth)acrylate | 2.208+/-0.228 | 2.756 +/- 0.254 |
| terbutyl(méth)acrylate | 2 22 +/- 0.238 | 2.574 +/- 0.261 |
| cyclohexyle (méth)acrylate | 2.853+/- 0.226 | 3.405 +/-0.252 |
| octyl(méth)acrylate | 4.513 ± 0.224 | 5 65 +/- 0.521 |
| lauryl(méth)acrylate | 6.638 ± 0.224 | 7.190 +/- 0.251 |
| tridecyl(méth)acrylate | 7.170 ± 0.224 | 7.712 ± 0.251 |
| cétyl (méth)acrylate | 8.764 ± 0.224 | 9.316 ± 0.251 |
| palmityl(méth)acrylate | >9 | >9 |
| stéaryl (méth)acrylate | 9.826 ± 0.224 | 10.379 ± 0.251 |
| behenyl (méth)acrylate | 12.504 ± 0.251 | 11.952 ± 0.225 |
| oléyl (méth)acrylate | 9.308 ± 0.232 | >9 |
| tétrahydrofurfuryl (méth)acrylate | 0,800 ± 0,263 | 1,352 ± 0,283 |
| 2-éthyl hexyl (méth)acrylate | 4,329 ± 0,229 | 4,881 ± 0,254 |
| 2-hydroxyéthyl (méth)acrylate | 0,166 ± 0,258 | 0,718 ± 0,277 |
| éthoxyéthyle (méth)acrylate | 1,335 ± 0,268 | 1,887 ± 0,293 |
| hydroxypropyl (méth)acrylate | 0,383 ± 0,241 | |
| diméthylaminoéthyle (méth)acrylate (MADAME) | | 0,97 |
| N-isopropyle (méth)acrylamide * | 0,195 ± 0,256 | 0,748 ± 0,276 |
| N-octyl (méth)acrylamide * | 3,036 ± 0,253 | 3,558 ± 0,273 |
| N-terbutyl (méth)acrylamide * | 1,02 | |
| N,N-diméthyl (méth)acrylamide* | -0,168 ± 0,556 | 0,906 ± 0,553 |
| N,N-dibutyl (méth)acrylamide* | 3,021 ± 0,557 | 3,573 ± 0,570 |
| acide (méth)acrylique | 0,35 | 0,83 |

Parmi les monomères de Tg inférieure ou égale à -20°C, susceptibles d'être employés pour former la dispersion selon l'invention, on peut citer :

- (i) les esters de l'acide acrylique de formule $CH_2$=CHCOOR1 avec R1 représentant (a) une chaîne carbonée, notamment hydrocarbonée (alkyle), ayant 2 à 12 atomes de carbone, linéaire ou ramifiée, saturée ou insaturée, comprenant éventuellement intercalé(s) un ou plusieurs hétéroatomes choisis parmi O, N, S; et/ou éventuellement substituée par un ou plusieurs substituants choisis parmi -OH et les atomes d'halogène (Cl, Br, I et F), à l'exclusion de la chaîne tertiobutyle; ou bien R1 représentant (b) un groupement polyoxyéthylène comprenant de 5 à 30 motifs d'oxyde d'éthylène; ou encore R1 représentant (c) un groupe -R-$(OC_2H_4)$n-H, avec R = alkyle en C1-C12 et n est

un entier compris entre 5 et 30 inclus.
On peut ainsi citer les acrylates d'éthyle, de propyle, de n-butyle, d'isobutyle, de 2-éthylhexyle, d'octyle, d'isooctyle, d'isodécyle, de décyle, de lauryle, de tridécyle, d'hydroxyéthyle et d'hydroxypropyle.

- (ii) les esters de l'acide méthacrylique de formule $CH_2=C(CH_3)COOR2$ avec R2 représentant (a) une chaîne carbonée, notamment hydrocarbonée (alkyle), ayant 8 à 12 atomes de carbone, linéaire ou ramifiée, saturée ou insaturée, comprenant éventuellement intercalé(s) un ou plusieurs hétéroatomes choisis parmi O, N, S; et/ou éventuellement substituée par un ou plusieurs substituants choisis parmi - OH et les atomes d'halogène (Cl, Br, I et F) ; ou bien R2 représentant (b) un groupement polyoxyéthylène comprenant de 5 à 30 motifs d'oxyde d'éthylène; ou encore R2 représentant (c) un groupe $-R-(OC_2H_4)n-H$, avec R = alkyle en C1-C30 et n est un entier compris entre 5 et 30 inclus.
  On peut ainsi citer les méthacrylates d'octyle, d'isooctyle, de décyle, d'isodécyle, de dodécyle, de lauryle, de tridécyle, de myristyle, de cétyle, de palmityle, de stéaryle, de béhényle, d'oléyle.

- (iii) les esters de vinyle de formule $CH_2=CH-OCO-R3$ avec R3 représentant une chaîne carbonée, notamment hydrocarbonée, ayant 2 à 12 atomes de carbone, linéaire ou ramifiée, saturée ou insaturée, parmi lesquels on peut citer le butyrate (ou butanoate) de vinyle, l'éthylhexanoate de vinyle, le néononanoate de vinyle et le néododécanoate de vinyle;

- (iv) les éthers de vinyle de formule $CH_2=CHOR4$ avec R4 représentant une chaîne carbonée, notamment hydrocarbonée, ayant 1 à 12 atomes de carbone, linéaire ou ramifiée, saturée ou insaturée; parmi lesquels on peut citer l'éther de vinyle, le méthylvinyléther, l'éthylvinyléther, l'éthylhexylvinyléther et le butylvinyléther;

- (v) les N-alkyl (méth)acrylamides de formule $CH_2=CHCONR5R'5$ ou $CH_2=C(CH_3)CONR5R'5$ avec R5 et R'5 représentant indépendamment l'un de l'autre, un atome d'hydrogène ou une chaîne carbonée, notamment hydrocarbonée, ayant 6 à 28 atomes de carbone, linéaire, cyclique ou ramifiée, saturée ou insaturée, éventuellement aromatique (aryle, aralkyle ou alkylaryle), comprenant éventuellement intercalé(s) un ou plusieurs hétéroatomes choisis parmi O, N, S; et/ou éventuellement substituée par un ou plusieurs substituants choisis parmi -OH et les atomes d'halogène (Cl, Br, I et F) ; étant donné que l'un au moins des radicaux R5, R'5 est différent de l'hydrogène; parmi lesquels on peut citer le N-octylacrylamide et le N-octadécylacrylamide.

Les monomères de Tg inférieure ou égale à -20°C plus particulièrement préférés sont l'acrylate de n-butyle, l'acrylate d'éthylhexyle, l'acrylate d'isobutyle, l'acrylate d'isooctyle et leur mélange.
Parmi les monomères ayant une Tg inférieure ou égale à -20°C et étant de plus hydrophobes, on peut citer les acrylates de n-butyle, d'isobutyle, de 2-éthylhexyle, d'octyle, d'isooctyle, d'isodécyle, de décyle, de lauryle; ainsi que les méthacrylates d'octyle, d'isooctyle, de décyle, d'isodécyle, de dodécyle, de lauryle, de tridécyle, de cétyle, de palmityle, de stéaryle, d'oléyle.
Encore plus préférentiellement, le monomère ayant une Tg inférieure ou égale à - 20 °C est l'acrylate de 2-éthyl hexyle.
Ainsi qu'il est mentionné plus haut, le polymère en dispersion selon l'invention peut comprendre un ou plusieurs monomères additionnels, de Tg supérieure à - 20°C, de préférence supérieure à 0°C, sous réserve que ce ou ces monomères additionnels, et/ou leur quantité, soient choisis de façon à ce que la Tg globale du polymère soit inférieure ou égale à -20°C.
Ces monomères additionnels peuvent être choisis parmi les monomères suivants :

- (i) les composés vinyliques de formule $CH_2=CHR6$ dans laquelle R6 est

  - un groupe hydroxyle;
  - un groupe alkyle linéaire ou ramifié, comprenant 1 à 25 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi -OH et les atomes d'halogène (Cl, Br, I et F);
  - un groupe cycloalkyle en $C_3$ à $C_8$ tel que cyclohexane,
  - un groupe aryle en $C_6$ à $C_{20}$ tel que phényle,
  - un groupe aralkyle en $C_7$ à $C_{30}$ (groupe alkyle en $C_1$ à $C_4$) tel que 2-phényléthyle ou benzyle,
  - un groupe hétérocyclique de 4 à 12 chaînons contenant un ou plusieurs hétéroatomes choisis parmi O, N, et S,
  - un groupe hétérocycloalkyle (alkyle de 1 à 4 carbones), tel que furfuryle, furfurylméthyle ou tétrahydrofurfurylméthyle,
    lesdits groupes cycloalkyle, aryle, aralkyle, hétérocyclique ou hétérocycloalkyle pouvant être éventuellement

substitué par un ou plusieurs substituants choisis parmi le groupe hydroxyle, les atomes d'halogène, et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi -OH, les atomes d'halogène (Cl, Br, I et F).
Des exemples de monomères vinyliques sont le vinylcyclohexane, le styrène et l'acétate de vinyle.

- (ii) les acrylates de formule $CH_2$=CHCOOR7 dans laquelle R7 est un groupe tertiobutyle, un groupe cycloalkyle en C3 à C8; un groupe aryle en C6 à C20 ; un groupe aralkyle en C7 à C30 (groupe alkyle en C1 à C4); un groupe hétérocyclique de 4 à 12 chaînons contenant un ou plusieurs hétéroatomes choisis parmi O, N et S; un groupe hétérocycloalkyle (alkyl de C1 à C4) tel qu'un groupe furfuryle; lesdits groupes cycloalkyle, aryle, aralkyle, hétérocyclique ou hétérocycloalkyle pouvant être éventuellement substitué par un ou plusieurs substituants choisis parmi le groupe hydroxyle, les atomes d'halogène et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi le groupe hydroxyle et les atomes d'halogène (Cl, Br, I et F).
  Des exemples de tels monomères sont les acrylates de tertio-butyle, de t-butylcyclohexyle, de t-butylbenzyle, de furfuryle et d'isobornyle.

- (iii) les méthacrylates de formule $CH_2=C(CH_3)$COOR8 dans laquelle R8 est :

  - un groupe carboné, notamment hydrocarboné (alkyle) ayant 1 à 6 atomes de carbone, linéaire ou ramifié, tel qu'un groupe méthyle, éthyle, propyle ou isobutyle, ledit groupe alkyle pouvant en outre être éventuellement substitué par un ou plusieurs substituants choisis parmi OH et les atomes d'halogène (Cl, Br, I et F) ;
  - un groupe cycloalkyle en C3 à C8 ;
  - un groupe aryle en C6 à C20 ;
  - un groupe aralkyle en C7 à C30 (groupe alkyle en C1 à C4) ;
  - un groupe hétérocyclique de 4 à 12 chaînons contenant un ou plusieurs hétéroatomes choisis parmi O, N et S;
  - un groupe hétérocycloalkyle (alkyle en C1-C4), tel qu'un groupe furfuryle ;
    lesdits groupes cycloalkyle, aryle, aralkyle, ou hétérocyclique ou hétérocycloalkyle pouvant être éventuellement substitué par un ou plusieurs substituants choisis parmi OH, les atomes d'halogène et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle et les atomes d'halogène (Cl, Br, I et F).
    Des exemples de tels monomères sont les méthacrylates de méthyle, d'éthyle, de propyle, de n-butyle, d'isobutyle, de t-butylcyclohexyle, de t-butylbenzyle, de méthoxyéthyle, de méthoxypropyle et d'isobornyle.
  - (iv) les (méth)acrylamides de formule $CH_2$=CHCONR9R'9 ou $CH_2=C(CH_3)$CONR9R'9 dans lesquelles R9 et R'9, identiques ou différents, représentent un atome d'hydrogène ou un groupe alkyle en C1-C5, linéaire ou ramifié, tel qu'un groupe n-butyle, t-butyle, isopropyle.
    Des exemples de tels monomères sont le N-butyl(méth)acrylamide, le N-isopropyl(méth)acrylamide, le N,N-diméthyl(méth)acrylamide et le N,N-dibutyl(méth)acrylamide.

Les monomères de Tg supérieure à -20°C plus particulièrement préférés sont les acrylates de furfuryle, d'isobornyle, de tertiobutyle, de tertiobutylcyclohexyle, de tertiobutylbenzyle; les méthacrylates de méthyle, de n-butyle, d'éthyle, d'isobutyle, le styrène, l'acétate de vinyle et le vinylcyclohexane, et leurs mélanges.
Parmi les monomères ayant une Tg supérieure à -20°C et étant de plus hydrophobes, on peut citer les acrylates d'isobornyle, de tertiobutyle, de tertiobutylcyclohexyle, de tertiobutylbenzyle; les méthacrylates de n-butyle, d'isobutyle, le styrène.
Le polymère présent dans la dispersion selon l'invention peut, bien évidemment, comprendre des monomères hydrophiles (c'est-à-dire non hydrophobes ou encore ayant un log P inférieur à 2), qui peuvent avoir une Tg inférieure ou égale à -20°C
ou une Tg supérieure à -20°C, ou un mélange de tels monomères hydrophiles.
Parmi les monomères hydrophiles susceptibles d'être employés, on peut citer l'acide acrylique, l'acide méthacrylique, le methacrylate de dimethylaminoethyle (MADAME), le méthacrylamide de diméthylaminopropyle (DMAPMA), la vinylpyridine et la vinylimidazole, le MADQUAT (ou [2-(methacryloyloxy)ethyl] trimethylammonium chloride), le MAPTAC (ou methacrylamidopropyltrimethylammonium chloride), le 2-hydroxyethylmethacrylate, le 2-hydroxyethylacrylate, le N-terbutylacrylamide, le tetrahydrofurfurylmethacrylate, le méthacrylate de méthyle, le méthacrylate d'éthyle, l'acrylate de méthyle, l'acrylate d'éthyle, la vinylpyrrolidone, le vinylacétate, les acrylates ou méthacrylates de formule CH2=C(H, $CH_3$)COOR avec R alkyle pouvant contenir des groupements (OC2H4)m-OR", avec m = 5 à 150 et R" = H ou alkyle de

C1 à C30.

De préférence, le monomère hydrophile est choisi parmi le méthacrylate de diméthylaminoéthyle.

Lorsqu'il comporte des fonction acides, le polymère selon l'invention peut être neutralisé par une base organique, par exemple une amine primaire, secondaire ou tertiaire, l'amine pouvant comporter ou non des substituants (hydroxyle), comme l'amino-2-méthyl-2-propanol, et les formes salifiées ou quaternisées de ceux-ci.

Lorsqu'il comporte des fonctions basiques, le polymère selon l'invention peut être neutralisé par des acides organiques qui peuvent comporter un ou plusieurs groupes carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyles. On peut notamment citer l'acide acétique, l'acide alpha-hydroxyéthanoïque, l'acide alpha-hydroxyoctanoïque, l'acide alpha-hydroxycaprylique, l'acide ascorbique, l'acide benzoïque, l'acide béhénique, l'acide caprique, l'acide caproïque, l'acide caprylique, l'acide citrique, l'acide dodécylbenzène sulfonique, l'acide 2-éthylcaproïque, l'acide folique, l'acide fumarique, l'acide galactarique, l'acide gluconique, l'acide glycolique, l'acide 2-hexadécyl eicosanoïque, l'acide hydroxycaproique, l'acide 12-hydroxystéarique, l'acide isolaurique (ou 2-butyl octanoïque), l'acide isomyristique (ou 2-hexyl octanoïque), l'acide isoarachidique (ou 2-octyl dodécanoïque), l'acide isolignocérique (ou 2-décyl tétradécanoïque), l'acide lactique, l'acide laurique, l'acide malique, l'acide myristique, l'acide oléïque, l'acide palmitique, l'acide propionique, l'acide sébacique, l'acide stéarique, l'acide tartrique, l'acide téréphtalique, l'acide trimésique, l'acide undécylénique, la propyle-bétaïne, la cocoamidopropylbétaïne, et leurs mélanges. De préférence, le polymère est neutralisé par un acide organique carboxylique et notamment les acides 2-éthylcaproïque, palmitique et décanoïque.

Les polymères utilisables dans le cadre de la présente invention ont de préférence un poids moléculaire moyen en nombre (Mn) compris entre 2000 à 1 000 0000, notamment entre 3000 et 800 000, et encore mieux entre 4000 et 500 000.

La dispersion de particules de polymères selon l'invention comprend donc un milieu non aqueux, dans lequel sont dispersées lesdites particules.

Ce milieu non aqueux est constitué d'au moins un composé non aqueux, liquide à 25°C, ayant un paramètre de solubilité global selon l'espace de solubilité de HANSEN inférieur ou égal à 20 $(MPa)^{1/2}$, ou un mélange de tels composés.

Le paramètre de solubilité global δ selon l'espace de solubilité de HANSEN est défini dans l'article "Solubility parameter values" de Grulke, dans l'ouvrage "Polymer Handbook" 3ème édition, Chapitre VII, pages 519-559 par la relation :

$$\delta = ( d_D^2 + d_P^2 + d_H^2)^{1/2}$$

dans laquelle :

- $d_D$ caractérise les forces de dispersion de LONDON issues de la formation de dipôles induits lors des chocs moléculaires,
- $d_P$ caractérise les forces d'interactions de DEBYE entre dipôles permanents,
- $d_H$ caractérise les forces d'interactions spécifiques (type liaisons hydrogène, acide/base, donneur/accepteur, etc.). La définition des solvants dans l'espace de solubilité tridimensionnel selon HANSEN est décrite dans l'article de HANSEN : "The three dimensionnal solubility parameters" J. Paint Technol. 39, 105 (1967).

Parmi les composés liquides non aqueux ayant un paramètre de solubilité global selon l'espace de solubilité de HANSEN inférieur ou égal à 20 $(MPa)^{1/2}$, on peut citer les corps gras liquides, notamment les huiles, qui peuvent être choisies parmi les huiles naturelles ou synthétiques, carbonées, hydrocarbonées, fluorées et/ou siliconées, éventuellement ramifiées, seules ou en mélange.

Parmi ces huiles, on peut citer les huiles végétales formées par des esters d'acides gras et de polyols, en particulier les triglycérides, telles que l'huile de tournesol, de sésame ou de colza, ou les esters dérivés d'acides ou d'alcools à longue chaîne (c'est à dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates, les myristates et les benzoates, notamment l'adipate de diisopropyle et le myristate d'isopropyle.

On peut également citer les hydrocarbures et notamment des huiles de paraffine, de vaseline, ou le polyisobutylène hydrogéné, les isoparaffines en $C_8$-$C_{16}$ et les isoparaffines volatiles tels que l'isododécane ou les 'ISOPARs'.

On peut encore citer les huiles de silicone telles que les polydiméthylsiloxanes et les polyméthylphénylsiloxanes, éventuellement substitués par des groupements aliphatiques et/ou aromatiques, éventuellement fluorés, ou par des

groupements fonctionnels tels que des groupements hydroxyles, thiols et/ou amines; et les huiles siliconées volatiles, notamment cycliques ou linéaires, telles que les cyclodiméthylsiloxanes, les cyclophénylmethylsiloxanes et les diméthylsiloxanes linéaires, parmi lesquels on peut citer la dodecamethylpentasiloxane linéaire (L5), l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane.

On peut également citer les solvants, seuls ou en mélange, choisis parmi les esters linéaires, ramifiés ou cycliques, ayant 6 à 30 atomes de carbone; les éthers ayant 6 à 30 atomes de carbone et les cétones ayant 6 à 30 atomes de carbone.

Parmi les composés non aqueux susceptibles d'être employés, on peut également citer les monoalcools ayant un paramètre de solubilité global selon l'espace de solubilité de HANSEN inférieur ou égal à 20 $(MPa)^{1/2}$, c'est-à-dire les monoalcools gras aliphatiques ayant au moins 6 atomes de carbone, notamment 6 à 32, la chaîne hydrocarbonée ne comportant pas de groupement de substitution. Comme monoalcools selon l'invention, on peut citer l'alcool oléique, le décanol, le dodécanol, l'octadécanol et l'alcool linoléique.

De préférence, le milieux non aqueux comprend des huiles siliconées volatiles, notamment cycliques ou linéaires, telles que les cyclodiméthylsiloxanes et les diméthylsiloxanes linéaires, et/ou des esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates, les myristates et les benzoates, notamment l'adipate de diisopropyle et le myristate d'isopropyle; ainsi que leurs mélanges.

Le choix du milieu non aqueux peut être effectué aisément par l'homme du métier en fonction de la nature des monomères constituant le polymère et/ou de la nature du stabilisant.

La dispersion de polymère peut être fabriquée comme décrit dans le document EP-A-749747. D'une manière générale, la polymérisation peut être effectuée en dispersion, c'est-à-dire par précipitation du polymère au cours de formation, avec protection des particules formées à l'aide d'un stabilisant.
On peut donc préparer un mélange comprenant les monomères initiaux ainsi qu'un amorceur radicalaire. Ce mélange est dissous dans un solvant de synthèse.
Les monomères sont solubles dans le milieu réactionnel, alors que le polymère n'y est pas soluble. Au fur et à mesure de la polymérisation, le polymère va précipiter et se trouver stabilisé par le stabilisant présent. On obtient ainsi des particules de polymères protégées en surface par le stabilisant.

On peut directement effectuer la polymérisation dans le milieu non aqueux qui peut donc jouer également le rôle de solvant de synthèse.
On peut également effectuer la polymérisation dans un solvant de synthèse, puis effectuer ensuite un échange de solvant, en remplaçant le solvant de synthèse par le milieu non aqueux.

Ainsi, lorsque le milieu non aqueux choisi est une huile non volatile, hydrocarbonée ou siliconée, on peut effectuer la polymérisation dans un solvant organique apolaire (solvant de synthèse) puis ajouter l'huile non volatile (qui doit être miscible avec ledit solvant de synthèse) et distiller sélectivement le solvant de synthèse.
On choisit donc de préférence un solvant de synthèse tel que les monomères initiaux, et l'amorceur radicalaire, y sont solubles, et les particules de polymère obtenu y sont insolubles afin qu'elles y précipitent lors de leur formation. En particulier, on peut choisir le solvant de synthèse parmi les alcanes tels que l'heptane, l'isododécane ou le cyclohexane.

Lorsque le milieu non aqueux choisi est une huile volatile, hydrocarbonée ou siliconée, on peut directement effectuer la polymérisation dans ladite huile qui joue donc également le rôle de solvant de synthèse. Les monomères doivent de préférence également y être solubles, ainsi que l'amorceur radicalaire, et le polymère obtenu doit y est insoluble.

Les monomères sont de préférence présents dans le solvant de synthèse, avant polymérisation, à raison de 5-80% en poids. La totalité des monomères peut être présente dans le solvant avant le début de la réaction, ou une partie des monomères peut être ajoutée au fur et à mesure de l'évolution de la réaction de polymérisation.
L'amorceur radicalaire peut être, par exemple, un composé azoïque ou peroxyde tels que l'azo-bis-isobutyronitrile ou le tertiobutylperoxy-2-éthyl hexanoate.

Les particules de polymère sont stabilisées en surface.
Dans un premier mode de réalisation, les particules peuvent être stabilisées en surface au fur et à mesure de la polymérisation, grâce à un stabilisant qui peut être notamment un polymère séquencé, un polymère greffé, et/ou un polymère statistique, seul ou en mélange. La stabilisation peut être effectuée par tout moyen connu, et en particulier par polymérisation en présence du stabilisant.
De préférence, le stabilisant est présent dans le mélange au départ de la polymérisation. Toutefois, il est également possible de l'ajouter en continu, notamment lorsqu'on ajoute également des monomères en continu.
Dans un second mode de réalisation, le polymère peut être synthétisé dans un solvant de synthèse, puis mis en dispersion dans un milieu non aqueux de dispersion par ajout du dispersant, et le solvant de synthèse évaporé.

On peut utiliser 0,1 à 30% en poids de stabilisant par rapport au poids du mélange initial de monomères, et de préférence de 1 à 20% en poids, préférentiellement de 2 à 15% en poids, voire de 3 à 10% en poids.

Lorsqu'on utilise un polymère greffé et/ou séquencé en tant que stabilisant, on choisit le solvant de synthèse de telle manière qu'au moins une partie des greffons
ou séquences dudit polymère-stabilisant soit soluble dans ledit solvant, l'autre partie des greffons ou séquences n'y étant pas soluble. Le polymère-stabilisant utilisé lors de la polymérisation doit être soluble, ou dispersible, dans le solvant de synthèse. De plus, on choisit de préférence un stabilisant comportant une partie (séquences, greffons ou autre), présentant une certaine affinité pour le polymère formé lors de la polymérisation.
Lorsqu'on utilise un polymère statistique en tant que stabilisant, on le choisit de manière à ce qu'il possède une quantité suffisante de groupements le rendant soluble dans le solvant de synthèse envisagé.
Parmi les polymères greffés, on peut citer les polymères siliconés greffés avec une chaîne hydrocarbonée et les polymères hydrocarbonés greffés avec une chaîne siliconée.
Conviennent également les copolymères greffés ayant par exemple un squelette insoluble de type polyacrylique avec des greffons solubles de type acide poly (12-hydroxy stéarique).
On peut également citer, comme polymère stabilisant :

- (a) les copolymères blocs greffés ou séquencés comprenant au moins un bloc de type polyorganosiloxane et au moins un bloc d'un polymère (i) issu de la polymérisation radicalaire ou (ii) issu d'une polycondensation, notamment de type polyéther, polyester ou polyamide, ou leur mélange, ledit copolymère pouvant comporter des entités fluorées;

Comme copolymères blocs greffés ou séquencés comprenant au moins un bloc de type polyorganosiloxane et au moins un bloc d'un polymère radicalaire, on peut citer les copolymères greffés de type acrylique/silicone qui peuvent être employés notamment lorsque le milieu non aqueux est siliconé.
Lorsque les copolymères blocs greffés ou séquencés comprennent au moins un bloc de type polyorganosiloxane et au moins un bloc polyéther, le bloc polyorganopolysiloxane peut être notamment un polydiméthylsiloxane ou bien encore un poly alkyl($C_2$-$C_{18}$) méthyl siloxane; le bloc polyéther peut être un poly alkylène en $C_2$-$C_{18}$, en particulier polyoxyéthylène et/ou polyoxypropylène. On peut ainsi utiliser les diméthicones copolyol ou encore les alkyl ($C_2$-$C_{18}$) méthicones copolyol, éventuellement réticulés. On peut par exemple utiliser le diméthicone copolyol vendu sous la dénomination "DOW CORNING 3225C" par la société DOW CORNING, ou le lauryl méthicone copolyol vendu sous la dénomination "DOW CORNING Q2-5200 par la société "DOW CORNING".
On peut aussi citer le lauryl diméthicone copolyol crosspolymer, par exemple le KSG31 ou KSG32 de Shin-Etsu, le cétyl diméthicone copolyol tel que le DMC 3071 de GE, et le diméthicone copolyol PPG-3-oléyl éther tel que le KF-6026 de Shin Etsu.

- (b) les copolymères blocs, greffés ou séquencés, de (méth)acrylates d'alkyle en
C1-C4 et de (méth)acrylates d'alkyle en C8-C30. On peut citer le copolymère méthacrylate de stéaryle/méthacrylate de méthyle.
- (c) les copolymères blocs greffés ou séquencés comprenant au moins un bloc résultant de la polymérisation de monomère éthylénique, à une ou plusieurs liaisons éthyléniques éventuellement conjuguées, et/ou notamment de diènes; et au moins un bloc de polymère issu de la polymérisation radicalaire autre que diène, notamment issu de monomère vinylique, (meth)acrylique ou (meth)acrylamide, ou d'un polyéther, d'un polyester ou d'un polyamide, ou leurs mélanges.
Notamment, on peut utiliser les copolymères comprenant au moins un bloc résultant de la polymérisation d'au moins un monomère éthylénique, à une ou plusieurs liaisons éthyléniques éventuellement conjuguées, tels que l'éthylène, le butadiène, l'isoprène, et d'au moins un bloc d'un polymère styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène. Parmi ces copolymères séquencés, on peut citer les copolymères de type "dibloc" ou "tribloc" du type polystyrène/polyisoprène, polystyrène/polybutadiène tels que ceux vendus sous le nom de 'LUVITOL HSB' par BASF, du type polystyrène/copoly(éthylène-propylène) tels que ceux vendus sous le nom de 'KRATON' par Shell Chemical Co ou encore du type polystyrène/copoly(éthylène-butylène).

Comme copolymères blocs greffés ou séquencés comprenant au moins un bloc résultant de la polymérisation d'au moins un monomère éthylénique, tels que l'éthylène, l'isobutylène, et d'au moins un bloc d'un polymère acrylique tel que le méthacrylate de méthyle, on peut citer les copolymères bi- ou triséquencés poly(méthacrylate de méthyle)/polyisobutylène ou les copolymères greffés à squelette poly(méthacrylate de méthyle) et à greffons polyisobutylène.
Comme copolymères blocs greffés ou séquencés comprenant au moins un bloc résultant de la polymérisation d'au moins un monomère éthylénique et d'au moins un bloc d'un polyéther tel qu'un polyoxyalkylène en C2-C18, en particulier polyoxyéthylène et/ou polyoxypropylène, on peut citer les copolymères bi- ou triséquencés polyoxyéthylène/polybutadiène ou polyoxyéthylène/polyisobutylène.

On peut également utiliser comme stabilisant, des composés tels que :

- (d) les alkyl-dimethicones dans lesquels le groupement alkyl comprend 6 à 32 atomes de carbone, tels que le lauryle methicone et le stéaryle methicone, notamment Si tec LDM 3107 d'ISP, le cetyl dimethicone tel que l'ABIL Wax 9801, le behenoxydimethicone tel que l'ABIL 5440 de Goldschmidt
- (e) les dimethiconol ester de formule :

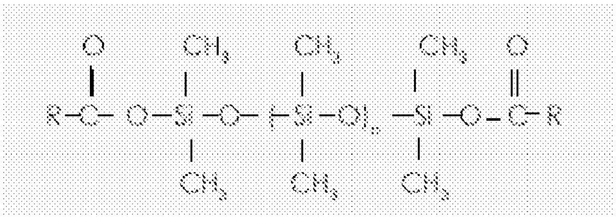

dans laquelle R est un radical alkyle ayant 6 à 32 atomes de carbone, tel que le dimethiconol behenate, et notamment les produits ULTRABEE de NOVEON et Pecosil DB de Phoenix Chemical.

- (f) les alkylamidoamines ayant notamment 6 à 60 atomes de carbone, notamment 12 à 50, telles que la behenamidopropyldimethylamine et notamment le Catemol 220 de Phoenix Chemical, de formule :

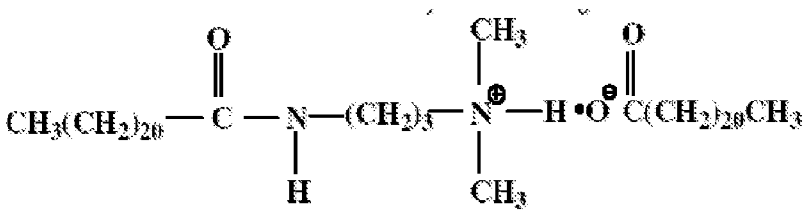

- (g) les copolymères comprenant au moins une partie polyorganosiloxane et des groupements fluorés, et notamment les silicones fluorées ou fluorosilicones qui peuvent être représentées par la formule :

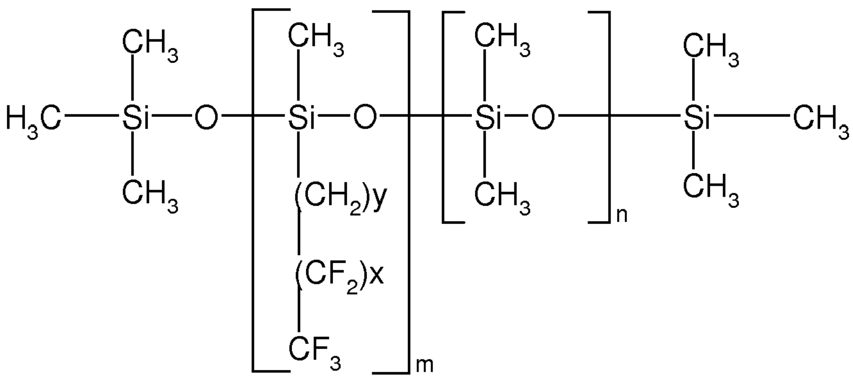

dans laquelle x est un entier compris entre 3 et 12, de préférence 5 et 10, notamment x=8; y est un entier compris entre 2 et 6, de préférence 2 ou 3; et m et n sont tels que le poids moléculaire du composé est compris entre 5000 et 15000; et plus particulièrement les perfluorononyldimethicone, tels que ceux vendus sous la dénomination PECOSIL FSH-150 et 300 ou PECOSIL FSL-150 et 300 par Phoenix Chemical;

Lorsque le solvant de synthèse est apolaire, il est préférable de choisir en tant que stabilisant, un polymère apportant une couverture des particules la plus complète possible, plusieurs chaînes de polymères-stabilisants venant alors s'adsorber sur une particule de polymère obtenu par polymérisation.

Dans ce cas, on préfère alors utiliser comme stabilisant, soit un polymère greffé, soit un polymère séquencé, de manière à avoir une meilleure activité interfaciale.

En effet, les séquences ou greffons insolubles dans le solvant de synthèse apportent une couverture plus volumineuse à la surface des particules.

Lorsque le solvant de synthèse liquide comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester comme les blocs polyoxypropyléné et/ou oxyéthyléné.

De manière particulièrement préférée, la dispersion selon l'invention est telle que :

- les monomères de Tg inférieure ou égale à -20°C, sont choisis parmi, seul ou en mélange, les acrylates d'éthyle, de propyle, de n-butyle, d'isobutyle, de 2-éthylhexyle, d'octyle, d'isooctyle, d'isodécyle, de décyle, de lauryle, de tridécyle, d'hydroxyéthyle et d'hydroxypropyle; les méthacrylates d'octyle, d'isooctyle, de décyle, d'isodécyle, de dodécyle, de lauryle, de tridécyle, de myristyle, de cétyle, de palmityle, de stéaryle, de béhényle, d'oléyle, et plus particulièrement comprend au moins de l'acrylate ou du méthacrylate d'éthyle 2-hexyle, et/ou
- les monomères de Tg inférieure ou égale à -20°C, représentent 50 à 100% en poids du poids total des monomères initiaux; et/ou
- l'agent stabilisant est choisi parmi les silicones fluorées ou fluorosilicones de formule :

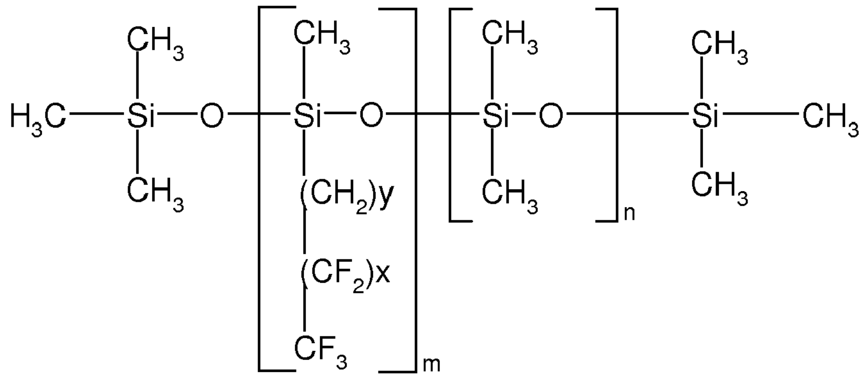

dans laquelle x=8, y = 2 ou 3, et m et n sont tels que le poids moléculaire du composé est compris entre 5000 et 15000; et/ou

- le composé liquide non aqueux est choisi parmi les huiles siliconées volatiles, notamment cycliques ou linéaires, telles que les cyclodiméthylsiloxanes et les diméthylsiloxanes linéaires, et/ou les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates, les myristates et les benzoates, notamment l'adipate de diisopropyle et le myristate d'isopropyle; ainsi que leurs mélanges.

Il est possible d'ajouter à la dispersion de polymères, un plastifiant de manière à abaisser la Tg des polymères utilisés. Le plastifiant peut être choisi parmi les plastifiants usuellement utilisés dans le domaine d'application et notamment parmi les composés susceptibles d'être des solvants du polymère. Le plastifiant peut être intégré lors de la synthèse ou ajouté une fois la synthèse réalisée.

Les polymères de la dispersion peuvent être utilisées dans les compositions cosmétiques selon l'invention à raison de 0,01 à 30% en poids de matière sèche, notamment de 0,1 à 20% en poids, voire de 0,1 à 10% en poids, encore mieux de 0,5 à 3% en poids, par rapport au poids total de la composition.

Les tensioactifs sont choisis parmi les tensioactifs anioniques, amphotères, non ioniques et leurs mélanges : <u>les tensioactifs anioniques</u> parmi lesquels on peut citer, seuls ou mélanges, les sels (en particulier sels de métaux alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulfonates, paraffine-sulfonates; les alkylsulfosuccinates, les alkyléthersulfosuccinates, les alkylamidesulfosuccinates; les alkylsulfosuccinamates; les alkylsulfoacétates ; les alkylétherphosphates; les acylsarcosinates; les acyliséthionates et les N-acyltaurates, le radical alkyle ou acyle de tous ces différents composés comportant de préférence de 8 à 24 atomes de carbone, et le radical aryle désignant de préférence un groupement phényle ou benzyle. On peut également citer les sels d'acides gras tels que les sels des acides oléique, ricinoléique, palmitique, stéarique, les acides d'huile de coprah ou d'huile de coprah hydrogénée; les acyl-lactylates dont le radical acyle comporte 8 à 20 atomes de carbone;

les acides d'alkyl D galactoside uroniques et leurs sels ainsi que les acides alkyl ($C_6$-$C_{24}$) éther carboxyliques polyoxyalkylénés, les acides alkyl($C_6$-$C_{24}$)aryl éther carboxyliques polyoxyalkylénés, les acides alkyl($C_6$-$C_{24}$) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser les alkylsufates, les alkyléthersulfates, les alkyléthercarboxylates, et leur mélanges, en particulier sous forme de sels de métaux alcalins ou alcalinaux terreux, d'ammonium, d'amine ou d'aminoalcool.

La quantité de tensioactifs anioniques va de préférence de 3% à 25% en poids, en particulier de 5% à 25% en poids, rapportée au poids total de la composition cosmétique.

- les tensioactifs non ioniques parmi lesquels on peut citer, seuls ou mélanges, les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C10-C14) amines ou les oxydes de N-acylaminopropylmorpholine.
  De préférnece, le tensioactif non ionique choisi parmi :
  - les alcools gras glycérolés ;
  - les alkylpolyglycosides.

Par chaîne grasse, on entend une chaîne hydrocarbonée comprenant 6 à 30 atomes de carbone, de préférence de 8 à 24 atomes de carbone, linéaire ou ramifiée, saturée ou non.

En ce qui concerne les alkypolyglycosides, ces composés sont bien connus et peuvent être plus particulièrement représenté par la formule générale suivante :

$$R_1O\text{-}(R_2O)_t\,(G)_v \qquad \text{(II)}$$

dans laquelle $R_1$ représente un radical alkyle et/ou alcényle linéaire ou ramifié comportant environ de 8 à 24 atomes de carbone, un radical alkylphényle dont le radical alkyle linéaire ou ramifié comporte de 8 à 24 atomes de carbone, $R_2$ représente un radical alkylène comportant environ de 2 à 4 atomes de carbone, G représente un motif sucre comportant de 5 à 6 atomes de carbone, t désigne une valeur allant de 0 à 10, de préférence 0 à 4, de préférence 0 à 4 et v désigne une valeur allant de 1 à 15.

Des alkylpolyglycosides préférés selon la présente invention sont des composés de formule (II) dans laquelle $R_1$ désigne plus particulièrement un radical alkyle saturé ou insaturé, linéaire ou ramifié comportant de 8 à 18 atomes de carbone, t désigne une valeur allant de 0 à 3 et plus particulièrement encore égale à 0, G peut désigner le glucose, le fructose ou le galactose, de préférence le glucose. Le degré de polymérisation, i.e. la valeur de v dans la formule (II), peut aller de 1 à 15, de préférence de 1 à 4. Le degré moyen de polymérisation est plus particulièrement compris entre 1 et 2 et encore plus préférenciellement de 1,1 à 1,5.
Les liaisons glycosidiques entre les motifs sucre sont de type 1-6 ou 1-4 et de préférence 1-4.

Des composés de formule (II) sont notamment représentés par les produits vendus par la société COGNIS sous les dénominations PLANTAREN® (600 CS/U, 1200 et 2000) ou PLANTACARE® (818, 1200 et 2000). On peut également utiliser les produits vendus par la société SEPPIC sous les dénominations TRITON CG 110 (ou ORAMIX CG 110) et TRITON CG 312 (ou ORAMIX® NS 10), les produits vendus par la société B.A.S.F. sous la dénomination LUTENSOL GD 70 ou encore ceux vendus par la société CHEM Y sous la dénomination AG10 LK.
On peut également utiliser par exemple, l'Alkyl en C8/C16 polyglucoside 1,4 en solution aqueuse à 53% commercialisé par COGNIS sous la référence PLANTACARE® 818 UP.

En ce qui concerne les tensioactifs mono ou polyglycérolés, ils comportent de préférence en moyenne de 1 à 30 groupements glycérol, plus particulièrement de 1 à 10 groupements glycérol et en particulier de 1,5 à 5.

Les tensioactifs monoglycérolés ou polyglycérolés sont de préférence choisis parmi les composés suivants :

A) les composés de formule suivantes : $RO[CH_2CH(CH_2OH)O]_mH$ $RO[CH_2CH(OH)CH_2O]_mH$ ou $RO[CH(CH_2OH)CH_2O]_mH$ ; dans laquelle R représente un radical hydrocarboné saturé ou insaturé, linéaire ou ramifié, comportant

de 8 à 40 atomes de carbone et de préférence de 10 à 30 atomes de carbone ; m est un nombre compris entre 1 et 30, de préférence entre 1 et 10, plus particulièrement de 1,5 à 6.
R peut éventuellement comprendre des hétéroatomes tels que par exemple oxygène et azote. En particulier, R peut éventuellement comprendre un ou plusieurs groupements hydroxy et/ou éther et/ou amide.
R désigne de préférence des radicaux alkyle et/ou alkényle en C10-C20, éventuellement mono ou polyhydroxylé.

On peut utiliser par exemple, l'hydroxylauryléther polyglycérolé (3.5 moles) commercialisé sous la dénomination Chimexane® NF de Chimex.
Parmi les tensioactifs non-ioniques on utilise de préférence les alkyl C6-C24 polyglucosides et plus particulièrement les alkyl C8-C16 polyglucoside.
La quantité totale de tensioactifs non ioniques va de préférence de 0.5% à 25% en poids, en particulier de 1% à 20% en poids, rapportée au poids total de la composition cosmétique et plus particulièrement de 2 à 10% en poids.

- <u>les tensioactifs amphotères</u> parmi lesquels on peut citer, seuls ou mélanges, les dérivés d'amines secondaires ou tertiaires aliphatiques, dans lesquels le radical aliphatique est une chaîne linéaire ou ramifiée comportant 8 à 22 atomes de carbone et comprenant au moins un groupe anionique hydrosolubilisant (par exemple carboxylate, sulfonate, sulfate, phosphate ou phosphonate); on peut citer encore les alkyl ($C_8$-$C_{20}$) bétaïnes, les sulfobétaïnes, les alkyl ($C_8$-$C_{20}$) amidoalkyl ($C_1$-$C_6$) bétaïnes telles que la cocoamidopropylbétaïne ou les alkyl ($C_8$-$C_{20}$) amidoalkyl ($C_1$-$C_6$) sulfobétaïnes.

Parmi les tensioactifs amphotères on utilise de préférence les alkyle C8-C20 bétaïnes, les alkyle C8-C20 amido alkyle C6-C8 bétaïnes, les alkylamphodiacétates et leur mélanges.
La quantité totale de tensioactifs amphotères va de préférence de 0.5% à 20% en poids, en particulier de 1% à 10% en poids, rapportée au poids total de la composition cosmétique et plus particulièrement de 1 à 5% en poids.
Parmi les mélanges de tensioactifs utilisables, on préfère les associations de tensioactif anioniques et de tensioactif amphotères et/ou non ioniques.
On utilise de préférence un agent tensioactif anionique choisi parmi les alkyl($C_{12}$-$C_{14}$) sulfates de sodium, de triéthanolamine ou d'ammonium, les alkyl ($C_{12}$-$C_{14}$)éthersulfates de sodium, de triéthanolamine ou d'ammonium oxyéthylénés à 2,2 moles d'oxyde d'éthylène, le cocoyl iséthionate de sodium et l'alphaoléfine($C_{14}$-$C_{16}$) sulfonate de sodium et leurs mélange avec :

- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocobétaïne commercialisée sous la dénomination "DEHYTON AB 30" en solution aqueuse à 32 % de MA par la société COGNIS.

La concentration totale des agents tensioactifs va de préférence de 4 % à 60% en poids, de préférence de 1% à 40% en poids, et encore plus préférentiellement de 5% à 30% en poids, par rapport au poids total de la composition.
La composition peut ainsi comprendre, un milieu hydrophile comprenant de l'eau
ou un mélange d'eau et de solvant(s) organique(s) hydrophile(s) comme les alcools et notamment les monoalcools, linéaires ou ramifiés en C1-C6, comme l'éthanol, le tertiobutanol, le n-butanol, l'isopropanol ou le n-propanol, et les polyols comme la glycérine, la diglycérine, le propylène glycol, le sorbitol, le pentylène glycol, et les polyéthylène glycols, ou bien encore les éthers de glycols notamment en $C_2$ et des cétones en $C_2$-$C_4$ hydrophiles.
L'eau ou le mélange d'eau et de solvants organiques hydrophiles peut être présent dans la composition selon l'invention en une teneur allant de 30% à 99% en poids, par rapport au poids total de la composition, et de préférence de 40% à 80% en poids.
La composition peut également comprendre une phase grasse, notamment constituée de corps gras liquides à température ambiante (25°C en général) et/ou de corps gras solides à température ambiante tels que les cires, les corps gras pâteux, les gommes et leurs mélanges. Ces corps gras peuvent être d'origine animale, végétale, minérale ou synthétique. Cette phase grasse peut, en outre, contenir des solvants organiques lipophiles.
Comme corps gras liquides à température ambiante, appelés souvent huiles, utilisables dans l'invention, on peut citer : les huiles hydrocarbonées d'origine animale telles que le perhydrosqualène; les huiles hydrocarbonées végétales telles que les triglycérides liquides d'acides gras de 4 à 10 atomes de carbone comme les triglycérides des acides heptanoïque ou octanoïque, ou encore l'huile d'amande douce, l'huile d'olive, l'huile de germes de blé, l'huile d'arachide, l'huile de colza, l'huile de carthame, l'huile de coprah, l'huile de noisette, l'huile de palme, l'huile de noyau d'abricot, l'huile de calophyllum, les huiles de tournesol, de maïs, de soja, de pépins de raisin, de sésame, de macadamia, de ricin, d'avocat,

les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol ; et les esters du pentaérythritol; des alcools gras ayant de 12 à 26 atomes de carbone comme l'octyldodécanol, le 2-butyloctanol, le 2-hexyldécanol, le 2-undécylpentadécanol, l'alcool oléique; les huiles fluorées partiellement hydrocarbonées et/ou siliconées; les huiles siliconées,; leurs mélanges.
Ces huiles peuvent être présentes en une teneur allant de 0,01 à 90%, et mieux de 0,1 à 85% en poids, par rapport au poids total de la composition.

Par cire au sens de la présente invention, on entend un composé lipophile, solide à température ambiante (25°C), à changement d'état solide/liquide réversible, ayant un point de fusion supérieur ou égal à 25°C pouvant aller jusqu'à 120°C. En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METLER.
Les cires peuvent être hydrocarbonées, fluorées et/ou siliconées et être d'origine végétale, minérale, animale et/ou synthétique. En particulier, les cires présentent un point de fusion supérieur à 30°C et mieux supérieur à 45°C. Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméthicone ayant de 16 à 45 atomes de carbone.
Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.
La nature et la quantité des corps solides sont fonction des propriétés mécaniques et des textures recherchées. A titre indicatif, la composition peut contenir de 0,1 à 50% en poids de cires, par rapport au poids total de la composition et mieux de 1 à 30% en poids.

La composition peut comprendre en outre un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques. Parmi les polymères filmogènes susceptibles d'être utilisés dans la composition de la présente invention, on peut citer les polymères synthétiques, de type radicalaire ou de type polycondensat, les polymères d'origine naturelle et leurs mélanges, en particulier les polymères acryliques, les polyuréthanes, les polyesters, les polyamides, les polyurées, les polymères cellulosiques comme la nitrocellulose.

La composition peut comprendre en outre un polymère conditionneur, généralement constitué par un polymère cationique Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse molaire moyenne en nombre ou en poids comprise entre 500 et $5.10^6$ environ, et de préférence comprise entre $10^3$ et $3.10^6$ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :

(1) les homopolymères ou copolymères dérivés d'esters ou d'amides acryliques ou méthacryliques et comportant au moins un des motifs de formules suivantes:

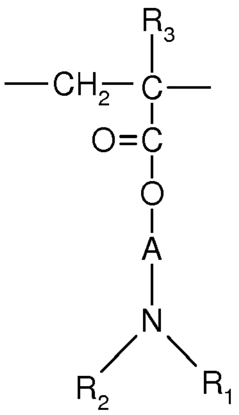

,

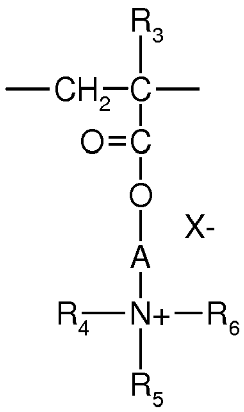

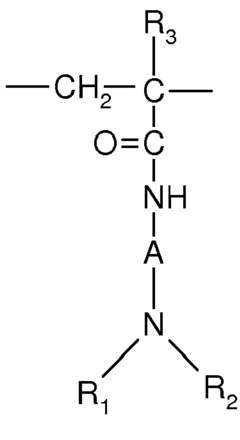

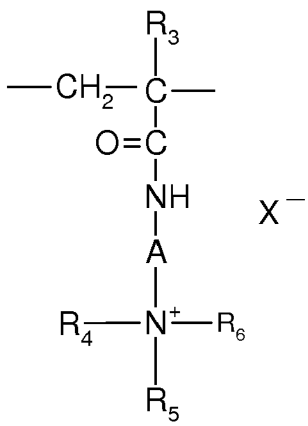

dans lesquelles:

$R_3$ , identiques ou différents, désignent un atome d'hydrogène ou un radical $CH_3$;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
$R_4$, $R_5$, $R_6$, identiques ou différents, représentent un groupe alkyle ayant de 1 à 18 atomes de carbone ou un radical benzyle et de préférence un groupe alkyle ayant de 1 à 6 atomes de carbone;
$R_1$ et $R_2$ , identiques ou différents, représentent hydrogène ou un groupe alkyle ayant de 1 à 6 atomes de carbone et de préférence méthyle ou éthyle;
X désigne un anion dérivé d'un acide minéral ou organique tel que un anion méthosulfate ou un halogénure tel que chlorure ou bromure.

Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.

Ainsi, parmi ces copolymères de la famille (1), on peut citer :

- les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
- les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
- le copolymères d'acrylamide et de méthosulfate de méthacryloyloxyéthyltriméthylammonium vendus sous la dénomination RETEN par la société HERCULES,
- les copolymères vinylpyrrolidone / acrylate ou méthacrylate de dialkylaminoalkyle quaternisés ou non. Ces polymères sont décrits en détail dans les brevets français 2 77.143 et 2.393.573,
- les terpolymères méthacrylate de diméthyl amino éthyle/ vinylcaprolactame/ vinylpyrrolidone,
- les copolymères vinylpyrrolidone / méthacrylamidopropyl dimethylamine.
- et les copolymères vinylpyrrolidone / méthacrylamide de diméthylaminopropyle quaternisé.

(2) Les polysaccharides cationiques notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.
Les dérivés d'éthers de cellulose comportant des groupements ammonium quaternaires décrits dans le brevet français 1 492 597. Ces polymères sont également définis dans le dictionnaire CTFA comme des ammonium quaternaires d'hydroxyéthylcellulose ayant réagi avec un époxyde substitué par un groupement triméthylammonium.
Les copolymères de cellulose cationiques ou les dérivés de cellulose greffés avec un monomère hydrosoluble d'ammonium quaternaire, sont décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.

Les gommes de galactomannane cationiques sont décrites plus particulièrement dans les brevets US 3 589 578 et 4 031 307 en particulier les gommes de guar contenant des groupements cationiques trialkylammonium. On utilise par exemple des gommes de guar modifiées par un sel (par ex. chlorure) de 2,3-époxypropyl triméthylammonium.

(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques
ou hétérocycliques, ainsi que les produits d'oxydation et/ou de quaternisation de ces polymères. De tels polymères sont notamment décrits dans les brevets français 2.162 25 et 2.280.361 ;

(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;

(5) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
Parmi ces dérivés, on peut citer plus particulièrement les polymères acide adipique/diméthylaminohydroxypropyl/diéthylène triamine vendus sous la dénomination "CARTARETINE F, F4 ou F8" par la société SANDOZ.

(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
Des polymères de ce type sont en particulier commercialisés sous la dénomination "HERCOSETT 57" par la société Hercules Inc. par la société HERCULES dans le cas du copolymère d'acide adipique/époxypropyl/diéthylène-triamine.

(7) les cyclopolymères d'alkyl diallyl amine ou de dialkyl diallyl ammonium tels que les homopolymères ou copolymères comportant comme constituant principal de la chaîne des motifs répondant aux formules (I) ou (I'):

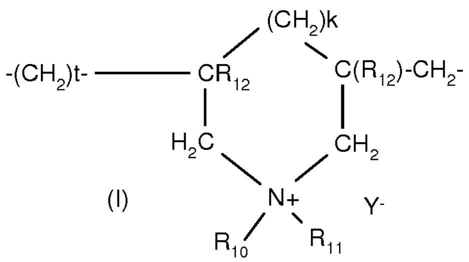

(I)

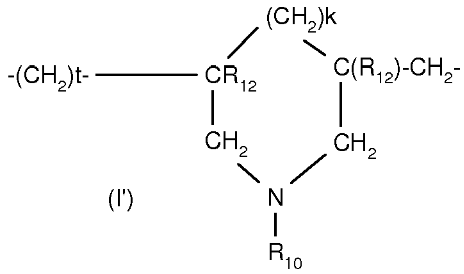

(I')

formules dans lesquelles k et t sont égaux à 0 ou 1, la somme k + t étant égale à 1 ; $R_{12}$ désigne un atome d'hydrogène ou un radical méthyle ; $R_{10}$ et $R_{11}$, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4) ou $R_{10}$ et $R_{11}$ peuvent désigner conjointement avec l'atome d'azote auquel ils sont rattachés, des groupement hétérocycliques, tels que pipéridinyle ou morpholinyle ; $Y^-$ est un anion tel que bromure, chlorure, acétate, borate, citrate, tartrate, bisulfate, bisulfite, sulfate, phosphate. Ces polymères sont notamment décrits dans le brevet français 2 80.759 et dans son certificat d'addition 2.190.406. $R_{10}$ et $R_{11}$, indépendamment l'un de l'autre, désignent de préférence un groupement alkyle ayant de 1 à 4 atomes de carbone.
Parmi les polymères définis ci-dessus, on peut citer plus particulièrement l'homopolymère de chlorure de diméthyldiallylammonium vendu sous la dénomination "MERQUAT 100" par la société NALCO (et ses homologues de faibles masses molaires moyenne en poids) et les copolymères de chlorure de diallyldiméthylammonium et d'acrylamide.

(8) les polymères de diammonium quaternaire contenant des motifs récurrents répondant à la formule :

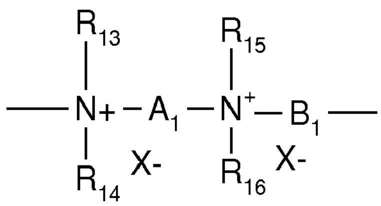

(II)

formule (II) dans laquelle :

$R_{13}$, $R_{14}$, $R_{15}$ et $R_{16}$, identiques ou différents, représentent des radicaux aliphatiques, alicycliques, ou arylaliphatiques contenant de 1 à 20 atomes de carbone ou des radicaux hydroxyalkylaliphatiques inférieurs, ou bien $R_{13}$, $R_{14}$, $R_{15}$ et $R_{16}$, ensemble ou séparément, constituent avec les atomes d'azote auxquels ils sont rattachés des hétérocycles contenant éventuellement un second hétéroatome autre que l'azote ou bien $R_{13}$, $R_{14}$, $R_{15}$ et $R_{16}$ représentent un radical alkyle en C1-C6 linéaire ou ramifié substitué par un groupement nitrile, ester, acyle, amide ou -CO-O-$R_{17}$-D ou -CO-NH-$R_{17}$-D où $R_{17}$ est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et $X^-$ désigne un anion dérivé d'un acide minéral ou organique;
A1, R13 et R15 peuvent former avec les deux atomes d'azote auxquels ils sont rattachés un cycle pipérazinique ; en outre si A1 désigne un radical alkylène

ou hydroxyalkylène linéaire ou ramifié, saturé ou insaturé, B1 peut également désigner un groupement (CH2)n-CO-D-OC-(CH2)n-
dans lequel D désigne :

a) un reste de glycol de formule : -O-Z-O-, où Z désigne un radical hydrocarboné linéaire ou ramifié ou un groupement répondant à l'une des formules suivantes :

- (CH2-CH2-O)x-CH2-CH2-
- [CH2-CH(CH3)-O]y-CH2-CH(CH3)-

où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

- CH2-CH2-S-S-CH2-CH2- ;

d) un groupement uréylène de formule : -NH-CO-NH- ;

De préférence, X- est un anion tel que le chlorure ou le bromure.
Ces polymères ont une masse molaire moyenne en nombre généralement comprise entre 1000 et 100000.
Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261 02, 2.271.378, 3.874.870, 4 01.432, 3.929.990, 3.966.904, 4 05.193, 4 25.617, 4 25.627, 4 25.653, 4 26.945 et 4 27 20.
On peut utiliser plus particulièrement les polymères qui sont constitués de motifs récurrents répondant à la formule :

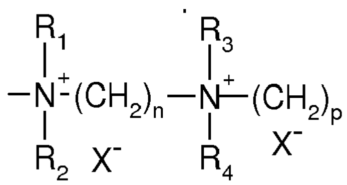

(a)

dans laquelle $R_1$, $R_2$, $R_3$ et $R_4$, identiques ou différents, désignent un radical alkyle ou hydroxyalkyle ayant de 1 à 4 atomes de carbone environ, n et p sont des nombres entiers variant de 2 à 20 environ et, $X^-$ est un anion dérivé d'un acide minéral ou organique.
Un composé de formule (a) particulièrement préféré est celui pour lequel $R_1$, $R_2$, $R_3$ et $R_4$, représentent un radical méthyle et n = 3, p = 6 et X = Cl, dénommé Hexadimethrine chloride selon la nomenclature INCI (CTFA).

(9) les polymères de polyammonium quaternaires comprenant des motifs de formule (III):

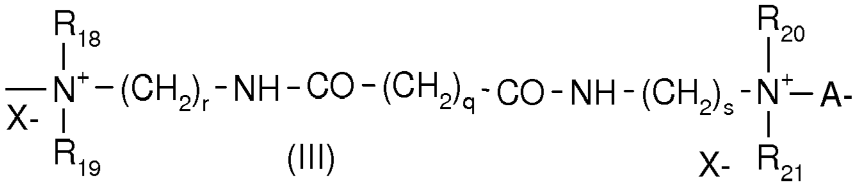

(III)

formule dans laquelle :

$R_{18}$, $R_{19}$, $R_{20}$ et $R_{21}$, identiques ou différents, représentent un atome d'hydrogène ou un radical méthyle, éthyle, propyle, β-hydroxyéthyle, β-hydroxypropyle ou $-CH_2CH_2(OCH_2CH_2)_pOH$,
où p est égal à 0 ou à un nombre entier compris entre 1 et 6, sous réserve que $R_{18}$, $R_{19}$, $R_{20}$ et $R_{21}$ ne représentent pas simultanément un atome d'hydrogène,
r et s, identiques ou différents, sont des nombres entiers compris entre 1 et 6,
q est égal à 0 ou à un nombre entier compris entre 1 et 34,
$X^-$ désigne un anion tel qu'un halogènure,
A désigne un radical d'un dihalogénure ou représente de préférence $-CH_2-CH_2-O-CH_2-CH_2-$.

De tels composés sont notamment décrits dans la demande de brevet EP-A-122 324.

On peut par exemple citer parmi ceux-ci, les produits "Mirapol® A 15", "Mirapol® AD1 ", "Mirapol® AZ1" et "Mirapol® 175" vendus par la société Miranol.

(10) Les polymères quaternaires de vinylpyrrolidone et de vinylimidazole tels que par exemple les produits commercialisés sous les dénominations Luviquat® FC 905, FC 550 et FC 370 par la société B.A.S.F.

(11) Les polymères réticulés de sels de méthacryloyloxyalkyl(C1-C4) trialkyl(C1-C4)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine, notamment les chitosanes ou leurs sels ;
les sels utilisables sont en particulier l'acétate, le lactate, le glutamate, le gluconate ou le pyrrolidone-carboxylate de chitosane.

Parmi ces composés, on peut citer le chitosane ayant un taux de désacétylation de 90 % en poids, le pyrrolidone-carboxylate de chitosane commercialisé sous la dénomination KYTAMER® PC par la société AMERCHOL.

Parmi tous les polymères cationiques susceptibles d'être utilisés dans le cadre de la présente invention, on préfère mettre en oeuvre les cyclopolymères cationiques, en particulier les homopolymères ou copolymères de chlorure de diméthyldiallylammonium, vendus sous les dénominations « MERQUAT 100 », « MERQUAT 550 » et « MERQUAT S » par la société NALCO, les polymères quaternaires de vinylpyrrolidone et de vinylimidazole, les homopolymères ou copolymères réticulés de sels de méthacryloyloxyalkyl($C_1$-$C_4$) trialkyl($C_1$-$C_4$)ammonium, , le pyrrolidone-carboxylate de chitosane commercialisé sous la dénomination KYTAMER® PC par la société AMERCHOL et leurs mélanges.

Selon l'invention, le ou les polymères cationiques ou amphotères peuvent représenter de 0,001 % à 20 % en poids, de préférence de 0,01 % à 10% en poids et plus particulièrement de 0,02 à 5% en poids par rapport au poids total de la composition finale.

Selon un mode préféré de l'invention, les compositions peuvent comprendre en outre au moins une silicone.

A titre de silicones utilisables dans les compositions de la présente invention, on peut notamment citer les silicones volatiles ou non, cycliques ou acycliques, ramifiées ou non, organomodifiées ou non, telles que décrites ci-dessous.
Les silicones utilisables conformément à l'invention peuvent être solubles ou insolubles dans la composition et en particulier être des polyorganosiloxanes insolubles dans la composition de l'invention ; elles peuvent se présenter sous forme d'huiles, de cires, de résines ou de gommes.
Selon l'invention, toutes les silicones peuvent être utilisées telle quelle ou sous formede solutions, de dispersions, d'émulsions, de nanoémulsions ou de micro-émulsions.
Les organopolysiloxanes sont définis plus en détail dans l'ouvrage de Walter NOLL "Chemistry and Technology of Silicones" (1968) Academie Press. Elles peuvent être volatiles ou non volatiles.
Lorsqu'elles sont volatiles, les silicones sont plus particulièrement choisies parmi celles possédant un point d'ébullition compris entre 60° C et 260° C, et plus particulièrement encore parmi :

(i) les silicones cycliques comportant de 3 à 7 atomes de silicium et, de préférence, 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.
On peut également citer les cyclocopolymères du type diméthylsiloxane/méthylalkylsiloxane, tel que la "SILICONE VOLATILE FZ 3109" commercialisée par la société UNION CARBIDE, de structure chimique :

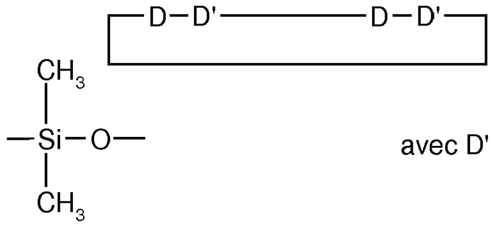

avec D :

avec D' : 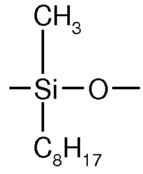

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bisnéopentane ;
(ii) les silicones volatiles linéaires ayant 2 à 9 atomes de silicium et possédant une viscosité inférieure ou égale à $5.10^{-6}m^2/s$ à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".
On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de $5.10^{-6}$ à 2,5 $m^2/s$ à 25°C et de préférence $1.10^{-5}$ à 1 $m^2/s$. La viscosité des silicones est, par exemple, mesurée à 25°C selon la norme ASTM 445 Appendice C.
Parmi ces polyalkylsiloxanes, on peut citer à titre non limitatif les produits commerciaux suivants :

- les huiles SILBIONE® des séries 47 et 70 047 ou les huiles MIRASIL® commercialisées par RHODIA telles que, par exemple, l'huile 70 047 V 500 000 ;
- les huiles de la série MIRASIL® commercialisées par la société RHODIA
- les huiles de la série 200 de la société DOW CORNING telles que plus particulièrement la DC200 de viscosité 60 000 $mm^2/s$ ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol, connus sous le nom de dimethiconol (CTFA), tels que les huiles de la série 48 de la société RHODIA.
Dans cette classe de polyalkylsiloxanes, on peut également citer les produits commercialisés sous les dénominations "ABIL WAX® 9800 et 9801" par la société GOLDSCHMIDT qui sont des polyalkyl($C_1$-$C_{20}$)-Siloxanes.
Les polyalkylarylsiloxanes sont particulièrement choisis parmi les polydiméthyl/méthylphénylsiloxanes, les polydiméthyldiphényl-siloxanes linéaires et/ou ramifiés de viscosité de $1.10^{-5}$ à $5.10^{-2}$ $m^2/s$ à 25°C.
Parmi ces polyalkylarylsiloxanes on peut citer, à titre d'exemple, les produits commercialisés sous les dénominations suivantes :

les huiles SILBIONE® de la série 70 641 de RHODIA ;
les huiles des séries RHODORSIL® 70 633 et 763 de RHODIA ;
. l'huile DOW CORNING 556 COSMETIC GRAD FLUID de DOW CORNING ;
. les silicones de la série PK de BAYER comme le produit PK20 ;
. les silicones des séries PN, PH de BAYER comme les produits PN1000 et PH1000 ;
. certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.
On peut plus particulièrement citer les produits suivants :

- polydiméthylsiloxane
- les gommes polydiméthylsiloxane/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :

- les mélanges formés à partir d'un polydiméthylsiloxane hydroxylé en bout de chaîne ou diméthiconol (CTFA) et d'un poly-diméthylsiloxane cyclique également appelé cyclométhicone (CTFA) tel que le produit Q2 1401 commercialisé par la société DOW CORNING ;
- les mélanges formés à partir d'une gomme polydiméthyl-siloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 $m^2/s$ et d'une huile SF 96 d'une viscosité de $5.10^{-6}$ $m^2/s$. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :
$R_2SiO_{2/2}$, $R_3SiO_{1/2}$, $RSiO_{3/2}$ et $SiO_{4/2}$ dans lesquels R représente un groupement hydrocarboné possédant 1 à 16 atomes de carbone ou un groupement phényle. Parmi ces produits, ceux particulièrement préférés sont ceux dans lesquels R désigne un groupe alkyle inférieur en $C_1$-$C_4$, plus particulièrement méthyle, ou un groupe phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthylsiloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :

- des groupements polyéthylèneoxy et/ou polypropylèneoxy comportant éventuellement des groupements alkyle en $C_6$-$C_{24}$ tels que les produits dénommés diméthicone-copolyol commercialisé par la société DOW CORNING sous la dénomination DC 1248 ou les huiles SILWET® L 722, L 7500, L 77, L 711 de la société UNION CARBIDE et l'alkyl($C_{12}$)-méthicone-copolyol commercialisée par la société DOW CORNING sous la dénomination Q2 5200 ;
- des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en $C_1$-$C_4$ ;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 .
- des groupements acyloxyalkyle tels que, par exemple, les polyorganosiloxanes décrits dans le brevet US-A-4957732.
- des groupements anioniques du type carboxylique comme, par exemple, dans les produits décrits dans le brevet EP 186 507 de la société CHISSO CORPORATION, ou de type alkyl-carboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL® S201" et "ABIL® S255".
- des groupements hydroxyacylamino, comme les polyorganosiloxanes décrits dans la demande EP 342 834. On peut citer, par exemple, le produit Q2-8413 de la société DOW CORNING.

Les silicones telles que décrites ci-dessus peuvent être utilisées seules ou en mélange, en une quantité allant

de 0,01 à 20 % en poids, de préférence de 0,1 à 5 % en poids par rapport au poids total de la composition.

La composition selon l'invention peut également comprendre des ingrédients couramment utilisés en cosmétique, tels que les vitamines, les parfums, les agents nacrants, les épaississants, les polymères différents des polymères de l'invention, les gélifiants, les oligo-éléments, les adoucissants, les séquestrants, les parfums, les agents alcalinisants ou acidifiants, les conservateurs, les filtres solaires, les anti-oxydants, les agents anti-chutes des cheveux, les agents anti-pelliculaires, les agents anti-gras, les huiles et les esters gras (en plus de ceux nécessaires à la dispersion des polymères), les anti-radicaux libres, les céramides, ou leurs mélanges. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Avantageusement, le pH de la composition de la présente invention est choisi dans la gamme allant de 2 à 11 et préférentiellement de 3 à 10 par exemple de 5 à 8.

La composition selon l'invention peut comprendre un propulseur. Le propulseur est choisi notamment parmi les gaz comprimés ou liquéfiés usuellement employés pour la préparation de compositions aérosols et leurs mélanges. On emploiera de manière préférentielle l'air, le gaz carbonique, l'azote comprimé ou encore un gaz soluble tel que le diméthyléther, les hydrocarbures halogénés (fluorés en particuliers) ou non et leurs mélanges.

Elle trouve notamment une application particulièrement intéressante dans le domaine capillaire, notamment pour le maintien de la coiffure ou la mise en forme des cheveux. Les compositions capillaires sont de préférence des shampooings, des gels, des lotions de mise en plis, des lotions pour le brushing, des compositions de fixation et de coiffage telles que les laques ou spray. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse.

Dans un mode de réalisation préféré, les compositions conformes à l'invention peuvent être utilisées pour le lavage et le traitement des matières kératiniques telles que les cheveux, la peau, les cils, les sourcils, les ongles, les lèvres, le cuir chevelu et plus particulièrement les cheveux.

Les compositions selon l'invention sont de préférence des compositions détergentes telles que des shampooings, des gels-douche et des bains moussants. Dans ce mode de réalisation de l'invention, les compositions comprennent au moins 4% en poids par rapport au poids total de la composition d'au moins un tensioactif détergent anionique et/ou non ionique.

L'invention a donc encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin et de préférence le lavage ou le démaquillage de la peau, des cheveux ou de toute autre matière kératinique.

Dans un autre mode de réalisation préféré, les compositions de l'invention peuvent se présenter sous forme d'après-shampooing à rincer ou non, ou encore sous forme de compositions à rincer, à appliquer avant ou aprèsa tout tritement capillaire, notamment une coloration, une décoloration, une permanente ou un défrisage ou encore entre les deux étapes d'une permanente ou d'un défrisage.

Lorsque la composition se présente sous la forme d'un après-shampooing éventuellement à rincer, elle contient avantageusement au moins un tensioactif cationique, par exemple en une concentration généralement comprise entre 0,1 et 10% en poids et de préférence de 0,5 à 5% en poids par rapport au poids total de la composition.

La composition suivant l'invention, après application sur les cheveux et cuir chevelu humains peut être rincée ou non rincée après tout traitement. Elle est de préférence rincée. Elle peut se présenter sous toute forme classiquement utilisée dans le domaine concerné et par exemple sous forme de lotion plus ou moins épaissie, de gel, de crème, de spray ou de mousse. Cette composition peut être monophasique ou multiphasique.

Selon un mode de réalisation préféré de l'invention, la composition peut être utilisée comme shampoing.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

Les compositions de l'invention sont illustrés plus en détail dans les exemples suivants.

<u>EXEMPLES de préparation des dispersions non aqueuses de particules de polymère</u>

*<u>Dispersion non aqueuse de polymère [1]</u>*

Dispersion de polyacrylate de 2 ethylhexyle dans cyclopentadimethylsiloxane. La dispersion est stabilisée par un polydimethylsiloxane à greffons perfluorononyle (PECOSIL FSH 150 commercialisé par la société PHOENIX°).

Dans un réacteur de 1 litre muni d'un réfrigérant et d'une ampoule d'addition de 250 ml, on introduit les constituants du pied de cuve. La température est fixée par régulation externe à 90°C, avec une montée en température de 2°C/minute, et une agitation mécanique de 300 tours/min. Une fois la température requise atteinte (90°C), on ajoute les constituants de la coulée, en 1 heure. On maintient la température à 90°C pendant 5 heures.

| | Pied de cuve (g) | Coulée (g) | Quantité totale (g) |
|---|---|---|---|
| Acrylate de 2-ethyle hexyle | 18 | 80 | 98 |
| PECOSI FSH-150 | 2 | -- | 2 |
| Huile de silicone (D5) | 90 | 90 | 180 |
| Amorceur (Trigonox 21S) | 2 | 0.6 | 2.6 |

On obtient une dispersion stable de polyacrylate d'éthyl-2 hexyle dans une huile siliconée volatile (D5). La Tg calculée du polymère est de -50°C.

La mesure de la taille moyenne en nombre des particules, effectuée par diffusion quasi-élastique de la lumière avec un Coulter N4 SD, donne une taille moyenne des particules de 150 nm.

Le poids moléculaire moyen en nombre, déterminé par GPC (solvant élution : THF) est de 19300 g/mol avec une polydispersité de 5.

*Dispersion non aqueuse de polymère [2]*

Dispersion de poly (acrylate de 2 ethylhexyle-codimethylaminoethylmethacrylate) neutralisé par de l'acide 2 ethylcarproïque dans de le myristate d'isopropyle. La dispersion est stabilisée un polydimethylsiloxane à greffons perfluorononyle (PECOSIL FSH 150 commercialisé par la société PHOENIX°).

Dans un réacteur de 500 ml muni d'un réfrigérant et d'une ampoule d'addition de 250 ml, on introduit les constituants du pied de cuve. La température est fixée par régulation externe à 90°C, avec une montée en température de 2°C/minute, et une agitation mécanique de 300 tours/min. Une fois la température requise atteinte (90°C), on ajoute les constituants de la coulée, en 1 heure et 20 minutes. On maintient la température à 90°C pendant 6 heures.

| | Pied de cuve (g) | Coulée (g) | Quantité totale (g) |
|---|---|---|---|
| Acrylate de 2-ethyle hexyle | -- | 70 | 70 |
| MADAME | -- | 30 | 30 |
| Acide éthylcaproïque | -- | 30 | 30 |
| PECOSIL FSH-150 | 2 | 3 | 5 |
| Myristate d'isopropyle | 50 | 150 | 200 |
| Amorceur (Trigonox 21S) | 0,5 | 1 | 1,5 |

On obtient une dispersion stable de particules de polyacrylate d'éthyl-2 hexyle/MADAME neutralisé à 100% dans le myristate d'isopropyle. La Tg estimée du polymère est inférieure à -33°C.

*Dispersion non aqueuse de polymère [3]*

Dispersion de poly (acrylate de 2 ethylhexyle-codimethylaminoethylmethacrylate) neutralisé par de l'acide décanoïque dans de le myristate d'isopropyle. La dispersion est stabilisée par un polydimethylsiloxane à greffons perfluorononyle (PECOSIL FSH 150 commercialisé par la société PHOENIX°).

Le même procédé de synthèse décrit dans l'exemple 2 est appliqué en remplaçant l'acide ethylcaproïque par de l'acide décanoïque.

*Dispersion non aqueuse de polymère [4]*

Dispersion de poly (acrylate de 2 ethylhexyle-codimethylaminoethylmethacrylate) neutralisé par de l'acide

palmitique dans de le myristate d'isopropyle. La dispersion est stabilisée par un polydimethylsiloxane à greffons perfluorononyle (PECOSIL FSH 150 commercialisé par la société PHOENIX°).

Le même procédé de synthèse décrit dans l'exemple 2 est appliqué en remplaçant l'acide ethylcaproique par de l'acide palmitique

**Exemples de shampooings selon l'invention**

L'invention peut être exemplifiée par les compositions non exhaustives suivantes.
Les pourcentages sont exprimés en pourcentage en poids de matière active.

| En gMA | 1 | 2 | 3 | 4 |
|---|---|---|---|---|
| Cocoglucoside (Plantacare 818 UP de COGNIS) | 10 | 10 | 10 | 10 |
| Cocoylbétaïne (DEHYTON AB 30 de GOLDSCHMIDT) | 3 | 3 | 3 | 3 |
| Cocamide MIPA (EMPILAN CIS de HUNTSMAN) | 2 | 2 | 2 | 2 |
| Carbomer (CARBOPOL 980 de NOVEON) | 0,3 | 0,3 | 0,3 | 0,3 |
| Dispersion non aqueuse de Polymère [1] | 0,5 | - | - | |
| Dispersion non aqueuse de Polymère [2] | - | 0,5 | | |
| Dispersion non aqueuse de Polymère [3] | | | 0,5 | |
| Dispersion non aqueuse de Polymère [4] | | | | 0,5 |
| Hexylèneglycol | 1 | 1 | 1 | 1 |
| NaCl | 1 | 1 | 1 | 1 |
| Conservateur | qs | qs | qs | qs |
| Parfum | qs | qs | qs | qs |
| Acide citrique/soude | qs pH 6,5 | qs pH 6,5 | qs pH 6,5 | qs pH 6,5 |
| Eau | qsp 100 | qsp 100 | qsp 100 | qsp 100 |

| En gMA | 5 | 6 | 7 | 8 |
|---|---|---|---|---|
| Sodium laureth sulfate<br>Texapon N 702 (COGNIS) | 12.5 | 12.5 | 12.5 | 12.5 |
| Coco betaine<br>Dehyton AB 30 (COGNIS) | 2.5 | 2.5 | 2.5 | 2.5 |
| Dispersion non aqueuse de polymère [1] | 1 | | - | - |
| Dispersion non aqueuse de polymère [2] | - | 1 | - | - |
| Dispersion non aqueuse de polymère [3] | - | - | 1 | |
| Dispersion non aqueuse de polymère [4] | - | - | | 1 |
| Hexylène glycol | 1 | 1 | 1 | 1 |
| Chlorure de sodium | 1 | 1 | 1 | 1 |
| Conservateur | qs | qs | qs | qs |
| Parfum | qs | qs | qs | qs |
| Acide citrique/soude | qs pH 6.5 | qs pH 6.5 | qs pH 6.5 | qs pH 6.5 |
| Eau | qsp 100 | qsp 100 | qsp 100 | qsp 100 |

| | 9 | 10 | 11 | 12 |
|---|---|---|---|---|
| Sodium laureth sulfate<br>Texapon N 702 (COGNIS) | 12,5 | 12,5 | 12,5 | 12,5 |
| Coco betaine<br>Dehydron AB 30 (COGNIS) | 2,5 | 2,5 | 2,5 | 2,5 |
| Polyquaternium-10<br>Ucare Polymer JR 400 (AMERCHOL) | 0,5 | 0,5 | 0,5 | 0,5 |
| Dispersion non aqueuse de polymère [1] | 1 | | - | - |
| Dispersion non aqueuse de polymère [2] | - | 1 | - | - |
| Dispersion non aqueuse de polymère [3] | - | - | 1 | |
| Dispersion non aqueuse de polymère [4] | - | - | | 1 |
| Hexylène glycol | 1 | 1 | 1 | 1 |
| Chlorure de sodium | 1 | 1 | 1 | 1 |
| Conservateur | qs | qs | qs | qs |
| Parfum | qs | qs | qs | qs |
| Acide citrique/soude | qs pH 6,5 | qs pH 6,5 | qs pH 6,5 | qs pH 6,5 |
| Eau | qsp 100 | qsp 100 | qsp 100 | qsp 100 |

| | 13 | 14 | 15 | 16 |
|---|---|---|---|---|
| Sodium laureth sulfate Texapon N 702 (COGNIS) | 12,5 | 12,5 | 12,5 | 12,5 |
| Coco betaine Dehydron AB 30 (COGNIS) | 2,5 | 2,5 | 2,5 | 2,5 |
| Cocamide MIPA Empilan CIS (HUNTSMAN) | 0,7 | 0,7 | 0,7 | 0,7 |
| Carbomer Carbomer 980 (NOVEON) | 0,3 | 0,3 | 0,3 | 0,3 |
| Dimethicone Mirasil DM 500 000 (RHODIA) | 2 | 2 | 2 | 2 |
| Guar hydroxypropyltrimonium chloride Jaguar C 13 S (RHODIA) | 0,1 | 0,1 | 0,1 | 0,1 |
| Dispersion non aqueuse de polymère [1] | 0,5 | | - | - |
| Dispersion non aqueuse de polymère [2] | - | 0,5 | - | - |
| Dispersion non aqueuse de polymère [3] | - | - | 0,5 | |
| Dispersion non aqueuse de polymère [4] | - | - | | 0,5 |
| Hexylène glycol | 1 | 1 | 1 | 1 |
| Chlorure de sodium | 0,5 | 0,5 | 0,5 | 0,5 |
| Conservateur | qs | qs | qs | qs |
| Parfum | qs | qs | qs | qs |
| Acide citrique/soude | qs pH 6,5 | qs pH 6,5 | qs pH 6,5 | qs pH 6,5 |
| Eau | qsp 100 | qsp 100 | qsp 100 | qsp 100 |

| En gMA | 17 | 18 | 19 | 20 |
|---|---|---|---|---|
| Sodium laureth sulfate Texapon N 702 (COGNIS) | 12,5 | 12,5 | 12,5 | 12,5 |
| Coco Glucoside Plantacare 818 UP (COGNIS) | 7,5 | 7,5 | 7,5 | 7,5 |
| Cocamide MIPA Empilan CIS (HUNTSMAN) | 1 | 1 | 1 | 1 |
| Carbomer Carbomer 980 (NOVEON) | 0,3 | 0,3 | 0,3 | 0,3 |
| Dispersion non aqueuse de polymère [1] | 0,5 | | - | - |
| Dispersion non aqueuse de polymère [2] | - | 0,5 | - | - |
| Dispersion non aqueuse de polymère [3] | - | - | 0,5 | |
| Dispersion non aqueuse de polymère [4] | - | - | | 0,5 |
| Hexylene glycol | 1 | 1 | 1 | 1 |
| Chlorure de sodium | 0,5 | 0,5 | 0,5 | 0,5 |
| Conservateur | qs | qs | qs | qs |
| Parfum | qs | qs | qs | qs |
| Acide citrique/soude | qs pH 6,5 | qs pH 6,5 | qs pH 6,5 | qs pH 6,5 |
| Eau | qsp 100 | qsp 100 | qsp 100 | qsp 100 |

| En gMA | 21 | 22 | 23 | 24 |
|---|---|---|---|---|
| Coco Glucoside<br>Plantacre 818 UP (COGNIS) | 10 | 10 | 10 | 10 |
| Coco betaine<br>Dehydron AB 30 (COGNIS) | 3 | 3 | 3 | 3 |
| Cocamide MIPA<br>Empilan CIS (HUNTSMAN) | 2 | 1 | 1 | 1 |
| Carbomer<br>Carbomer 980 (NOVEON) | 0,3 | 0,3 | 0,3 | 0,3 |
| Dispersion non aqueuse de polymère [1] | 0,5 | | - | - |
| Dispersion non aqueuse de polymère [2] | - | 0,5 | - | - |
| Dispersion non aqueuse de polymère [3] | - | - | 0,5 | |
| Dispersion non aqueuse de polymère [4] | - | - | | 0,5 |
| Hexylene glycol | 1 | 1 | 1 | 1 |
| Chlorure de sodium | 0,5 | 0,5 | 0,5 | 0,5 |
| Conservateur | qs | qs | qs | qs |
| Parfum | qs | qs | qs | qs |
| Acide citrique/soude | qs pH 6,5 | qs pH 6,5 | qs pH 6,5 | qs pH 6,5 |
| Eau | qsp 100 | qsp 100 | qsp 100 | qsp 100 |

Les cheveux traités avec les compositions selon l'invention présentent, une fois la chevelure séchée une bonne tenue et une mise ne forme particulièrement aisée.

## Revendications

1. Composition cosmétique **caractérisée par le fait qu'**elle comprend dans un milieu aqueux cosmétiquement acceptable:

   I) au moins un tensioactif anionique, amphotère, non ionique, et leurs mélanges
   II) au moins une dispersion de particules d'au moins un polymère éthylénique stabilisé en surface par un agent stabilisant, dans un milieu non aqueux constitué d'au moins un composé non aqueux, liquide à 25°C, ayant un paramètre de solubilité global selon l'espace de solubilité de HANSEN inférieur ou égal à 20 $(MPa)^{1/2}$, ou un mélange de tels composés;
   ledit polymère éthylénique présentant une température de transition vitreuse (Tg) inférieure ou égale à -20°C.

2. Composition selon la revendication 1, dans laquelle le polymère éthylénique présente une température de transition vitreuse (Tg) comprise entre -150°C et - 20°C, notamment entre -100°C et -25°C, préférentiellement entre -95°C et -30°C, voire entre -80°C et -35°C, et encore mieux entre -70°C et -40°C.

3. Composition selon l'une des revendications précédentes, **caractérisée en ce que** la dispersion se présente sous forme de nanoparticules de polymère en dispersion dans le milieu non aqueux, lesdites nanoparticules ayant une taille comprise entre 5 et 600 nm, notamment 10 à 500 nm, encore mieux 15 à 450 nm.

4. Composition selon l'une des revendications précédentes, **caractérisée en ce que** le polymère est issu de la polymérisation d'un ou plusieurs monomères de Tg inférieure ou égale à -20°C, qui représente(nt) 100% en poids du poids total des monomères.

5. Composition selon l'une des revendications 1 à 3, **caractérisée en ce que** le polymère est issu de la polymérisation d'un ou plusieurs monomères de Tg inférieure ou égale à -20°C, et d'un ou plusieurs monomères additionnels de Tg supérieure à -20°C.

**6.** Composition selon la revendication 5, **caractérisée en ce que** le monomère additionnel, ou le mélange de tels monomères, est présent à raison de 0,01 à 50% en poids, par rapport au poids total des monomères, notamment de 0,1 à 40% en poids, voire de 1 à 30% en poids, ou encore de 5 à 15% en poids; et le monomère de Tg inférieure ou égale à -20°C, ou le mélange de tels monomères, est présent à raison de 50 à 99,99% en poids, notamment de 60 à 99,9% en poids, voire de 70 à 99% en poids, ou encore de 85 à 95% en poids, par rapport au poids total de monomères.

**7.** Composition selon l'une des revendications précédentes, **caractérisée en ce que** le polymère éthylénique comprend 40% à 100% en poids, par rapport au poids total de monomères, notamment de 60 à 99% en poids, voire de 70 à 98% poids, et encore mieux de 60 à 95% en poids de monomère hydrophobe, seul ou en mélange.

**8.** Composition selon la revendication 7, dans laquelle le monomère hydrophobe a une Tg inférieure à -20°C.

**9.** Composition selon l'une des revendications 4 à 6, dans laquelle les monomères de Tg inférieure ou égale à -20°C, sont choisis parmi, seul ou en mélange :

- (i) les esters de l'acide acrylique de formule $CH_2$=CHCOOR1 avec R1 représentant (a) une chaîne carbonée, notamment hydrocarbonée (alkyle), ayant 2 à 12 atomes de carbone, linéaire ou ramifiée, saturée ou insaturée, comprenant éventuellement intercalé(s) un ou plusieurs hétéroatomes choisis parmi O, N, S; et/ou éventuellement substituée par un ou plusieurs substituants choisis parmi -OH et les atomes d'halogène (Cl, Br, I et F), à l'exclusion de la chaîne tertiobutyle; ou bien R1 représentant (b) un groupement polyoxyéthylène comprenant de 5 à 30 motifs d'oxyde d'éthylène; ou encore R1 représentant (c) un groupe -R-($OC_2H_4$)n-H, avec R = alkyle en C1-C12 et n est un entier compris entre 5 et 30 inclus;
- (ii) les esters de l'acide méthacrylique de formule $CH_2$=C($CH_3$)COOR2 avec R2 représentant (a) une chaîne carbonée, notamment hydrocarbonée (alkyle), ayant 8 à 12 atomes de carbone, linéaire ou ramifiée, saturée ou insaturée, comprenant éventuellement intercalé(s) un ou plusieurs hétéroatomes choisis parmi O, N, S; et/ou éventuellement substituée par un ou plusieurs substituants choisis parmi - OH et les atomes d'halogène (Cl, Br, I et F) ; ou bien R2 représentant (b) un groupement polyoxyéthylène comprenant de 5 à 30 motifs d'oxyde d'éthylène; ou encore R2 représentant (c) un groupe -R-($OC_2H_4$)n-H, avec R = alkyle en C1-C30 et n est un entier compris entre 5 et 30 inclus;
- (iii) les esters de vinyle de formule $CH_2$=CH-OCO-R3 avec R3 représentant une chaîne carbonée, notamment hydrocarbonée, ayant 2 à 12 atomes de carbone, linéaire ou ramifiée, saturée ou insaturée;
- (iv) les éthers de vinyle de formule $CH_2$=CHOR4 avec R4 représentant une chaîne carbonée, notamment hydrocarbonée, ayant 1 à 12 atomes de carbone, linéaire ou ramifiée, saturée ou insaturée;
- (v) les N-alkyl (méth)acrylamides de formule $CH_2$=CHCONR5R'5 ou $CH_2$=C($CH_3$)CONR5R'5 avec R5 et R'5 représentant indépendamment l'un de l'autre, un atome d'hydrogène ou une chaîne carbonée, notamment hydrocarbonée, ayant 6 à 28 atomes de carbone, linéaire, cyclique ou ramifiée, saturée ou insaturée, éventuellement aromatique (aryle, aralkyle ou alkylaryle), comprenant éventuellement intercalé(s) un ou plusieurs hétéroatomes choisis parmi O, N, S; et/ou éventuellement substituée par un ou plusieurs substituants choisis parmi -OH et les atomes d'halogène (Cl, Br, I et F) ; étant donné que l'un au moins des radicaux R5, R'5 est différent de l'hydrogène.

**10.** Composition selon la revendication 9, dans laquelle les monomères de Tg inférieure ou égale à -20°C, sont choisis parmi, seul ou en mélange :

- les acrylates d'éthyle, de propyle, de n-butyle, d'isobutyle, de 2-éthylhexyle, d'octyle, d'isooctyle, d'isodécyle, de décyle, de lauryle, de tridécyle, d'hydroxyéthyle et d'hydroxypropyle.
- les méthacrylates d'octyle, d'isooctyle, de décyle, d'isodécyle, de dodécyle, de lauryle, de tridécyle, de myristyle, de cétyle, de palmityle, de stéaryle, de béhényle, d'oléyle.
- le butyrate (ou butanoate) de vinyle, l'éthylhexanoate de vinyle, le néononanoate de vinyle et le néododécanoate de vinyle.
- l'éther de vinyle, le méthylvinyléther, l'éthylvinyléther, l'éthylhexylvinyléther et le butylvinyléther;
- le N-octylacrylamide et le N-octadécylacrylamide.

**11.** Composition selon la revendication 9, dans laquelle les monomères de Tg inférieure ou égale à -20°C, sont choisis parmi les acrylates de n-butyle, d'isobutyle, de 2-éthylhexyle, d'octyle, d'isooctyle, d'isodécyle, de décyle, de lauryle; ainsi que les méthacrylates d'octyle, d'isooctyle, de décyle, d'isodécyle, de dodécyle, de lauryle, de tridécyle, de cétyle, de palmityle, de stéaryle, d'oléyle; et leurs mélanges.

**12.** Composition selon la revendication 11, dans laquelle le monomères de Tg inférieure ou égale à -20°C est l'acrylate de 2-éthylehexyle.

**13.** Composition selon l'une des revendications 5 à 6 ou 9 à 12, dans laquelle les monomères additionnels sont choisis parmi, seul ou en mélange :

- (i) les composés vinyliques de formule $CH_2$=CHR6 dans laquelle R6 est

    - un groupe hydroxyle;
    - un groupe alkyle linéaire ou ramifié, comprenant 1 à 25 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi -OH et les atomes d'halogène (Cl, Br, I et F);
    - un groupe cycloalkyle en $C_3$ à $C_8$ tel que cyclohexane,
    - un groupe aryle en $C_6$ à $C_{20}$ tel que phényle,
    - un groupe aralkyle en $C_7$ à $C_{30}$ (groupe alkyle en $C_1$ à $C_4$) tel que 2-phényléthyle ou benzyle,
    - un groupe hétérocyclique de 4 à 12 chaînons contenant un ou plusieurs hétéroatomes choisis parmi O, N, et S,
    - un groupe hétérocycloalkyle (alkyle de 1 à 4 carbones), tel que furfuryle, furfurylméthyle ou tétrahydrofurfurylméthyle,

    lesdits groupes cycloalkyle, aryle, aralkyle, hétérocyclique ou hétérocycloalkyle pouvant être éventuellement substitué par un ou plusieurs substituants choisis parmi le groupe hydroxyle, les atomes d'halogène, et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi -OH, les atomes d'halogène (Cl, Br, I et F).

- (ii) les acrylates de formule $CH_2$=CHCOOR7 dans laquelle R7 est un groupe tertiobutyle, un groupe cycloalkyle en C3 à C8; un groupe aryle en C6 à C20 ; un groupe aralkyle en C7 à C30 (groupe alkyle en C1 à C4); un groupe hétérocyclique de 4 à 12 chaînons contenant un ou plusieurs hétéroatomes choisis parmi O, N et S; un groupe hétérocycloalkyle (alkyl de C1 à C4) tel qu'un groupe furfuryle; lesdits groupes cycloalkyle, aryle, aralkyle, hétérocyclique ou hétérocycloalkyle pouvant être éventuellement substitué par un ou plusieurs substituants choisis parmi le groupe hydroxyle, les atomes d'halogène et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi le groupe hydroxyle et les atomes d'halogène (Cl, Br, I et F).
- (iii) les méthacrylates de formule $CH_2$=C($CH_3$)COOR8 dans laquelle R8 est :

    - un groupe carboné, notamment hydrocarboné (alkyle) ayant 1 à 6 atomes de carbone, linéaire ou ramifié, tel qu'un groupe méthyle, éthyle, propyle ou isobutyle, ledit groupe alkyle pouvant en outre être éventuellement substitué par un ou plusieurs substituants choisis parmi OH et les atomes d'halogène (Cl, Br, I et F);
    - un groupe cycloalkyle en C3 à C8 ;
    - un groupe aryle en C6 à C20 ;
    - un groupe aralkyle en C7 à C30 (groupe alkyle en C1 à C4) ;
    - un groupe hétérocyclique de 4 à 12 chaînons contenant un ou plusieurs hétéroatomes choisis parmi O, N et S;
    - un groupe hétérocycloalkyle (alkyle en C1-C4), tel qu'un groupe furfuryle ;

    lesdits groupes cycloalkyle, aryle, aralkyle, ou hétérocyclique ou hétérocycloalkyle pouvant être éventuellement substitué par un ou plusieurs substituants choisis parmi OH, les atomes d'halogène et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle et les atomes d'halogène (Cl, Br, I et F).

- (iv) les (méth)acrylamides de formule $CH_2$=CHCONR9R'9 ou $CH_2$=C($CH_3$)CONR9R'9 dans lesquelles R9 et R'9, identiques ou différents, représentent un atome d'hydrogène ou un groupe alkyle en C1-C5, linéaire ou ramifié, tel qu'un groupe n-butyle, t-butyle, isopropyle.

**14.** Composition selon la revendication 13, dans laquelle les monomères additionnels sont choisis parmi le vinylcyclohexane, le styrène et l'acétate de vinyle; les acrylates de tertiobutyle, de t-butylcyclohexyle, de t-butylbenzyle, de furfuryle et d'isobornyle; les méthacrylates de méthyle, d'éthyle, de propyle, de n-butyle, d'isobutyle, de t-butylcyclohexyle, de t-butylbenzyle, de méthoxyéthyle, de méthoxypropyle et d'isobornyle; le N-butyl(méth)acrylamide, le N-isopropyl(méth)acrylamide, le N,N-diméthyl(méth)acrylamide et le N,N-dibutyl(méth)acrylamide, et leurs mélanges.

**15.** Composition selon l'une des revendications précédentes, dans laquelle le polymère éthylénique a un poids moléculaire moyen en nombre (Mn) compris entre 2000 à 1 000 0000, notamment entre 3000 et 800 000, et encore mieux entre 4000 et 500 000.

**16.** Composition selon l'une des revendications précédentes, dans laquelle ledit composé liquide non aqueux ayant un paramètre de solubilité global selon l'espace de solubilité de HANSEN inférieur ou égal à 20 $(MPa)^{1/2}$, est choisi parmi, seul ou en mélange, les corps gras liquides, notamment les huiles naturelles ou synthétiques, carbonées, hydrocarbonées, fluorées et/ou siliconées, éventuellement ramifiées.

**17.** Composition selon l'une des revendications précédentes, dans laquelle ledit composé liquide non aqueux est choisi parmi :

- les huiles végétales formées par des esters d'acides gras et de polyols, en particulier les triglycérides, telles que l'huile de tournesol, de sésame ou de colza, ou les esters dérivés d'acides ou d'alcools à longue chaîne (c'est à dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates, les myristates et les benzoates, notamment l'adipate de diisopropyle et le myristate d'isopropyle.
- les hydrocarbures et notamment des huiles de paraffine, de vaseline, ou le polyisobutylène hydrogéné, les isoparaffines en $C_8$-$C_{16}$ et les isoparaffines volatiles tels que l'isododécane ou les 'ISOPARs'.
- les huiles de silicone telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyles, thiols et/ou amines; et les huiles siliconées volatiles, notamment cycliques ou linéaires, telles que les cyclodiméthylsiloxanes, les cyclophénylmethylsiloxanes et les diméthylsiloxanes linéaires, parmi lesquels on peut citer la dodecamethylpentasiloxane linéaire (L5), l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane.
- les solvants, seuls ou en mélange, choisis parmi les esters linéaires, ramifiés ou cycliques, ayant 6 à 30 atomes de carbone; les éthers ayant 6 à 30 atomes de carbone et les cétones ayant 6 à 30 atomes de carbone.
- les monoalcools gras aliphatiques ayant au moins 6, notamment 6 à 32, atomes de carbone, la chaîne hydrocarbonée ne comportant pas de groupement de substitution, tels que l'alcool oléique, le décanol, le dodécanol, l'octadécanol et l'alcool linoléique.

**18.** Composition selon l'une des revendications précédentes, dans laquelle ledit composé liquide non aqueux est choisi parmi les huiles siliconées volatiles, notamment cycliques ou linéaires, telles que les cyclodiméthylsiloxanes et les diméthylsiloxanes linéaires, et/ou des esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates, les myristates et les benzoates, notamment l'adipate de diisopropyle et le myristate d'isopropyle; ainsi que leurs mélanges.

**19.** Composition selon l'une des revendications précédentes, dans laquelle l'agent stabilisant est choisi parmi les polymères séquencés, les polymères greffés et/ou les polymères statistiques, seul ou en mélange.

**20.** Composition selon l'une des revendications précédentes, dans laquelle l'agent stabilisant est présent à raison de 0,1 à 30% en poids par rapport au poids du mélange initial de monomères, de préférence de 1 à 20% en poids, préférentiellement de 2 à 15% en poids, voire de 3 à 10% en poids.

**21.** Composition selon l'une des revendications précédentes, dans laquelle l'agent stabilisant est choisi parmi :

- les polymères siliconés greffés avec une chaîne hydrocarbonée et les polymères hydrocarbonés greffés avec une chaîne siliconée.

- les copolymères greffés ayant un squelette insoluble de type polyacrylique avec des greffons solubles de type acide poly (12-hydroxy stéarique).
- les copolymères blocs greffés ou séquencés comprenant au moins un bloc de type polyorganosiloxane et au moins un bloc d'un polymère (i) issu de la polymérisation radicalaire ou (ii) issu d'une polycondensation, notamment de type polyéther, polyester ou polyamide, ou leur mélange, ledit copolymère pouvant comporter des entités fluorées; et notamment les copolymères greffés de type acrylique/silicone;
- les copolymères blocs, greffés ou séquences, de (méth)acrylates d'alkyle en C1-C4 et de (méth)acrylates d'alkyle en C8-C30; et notamment le copolymère méthacrylate de stéaryle/méthacrylate de méthyle.
- les copolymères blocs greffés ou séquences comprenant au moins un bloc résultant de la polymérisation de monomère éthylénique, à une ou plusieurs liaisons éthyléniques éventuellement conjuguées, et/ou notamment de diènes; et au moins un bloc de polymère issu de la polymérisation radicalaire autre que diène, notamment issu de monomère vinylique, (meth)acrylique ou (meth)acrylamide, ou d'un polyéther, d'un polyester ou d'un polyamide, ou leurs mélanges.
- les alkyl-dimethicones dans lesquels le groupement alkyl comprend 6 à 32 atomes de carbone, tels que le lauryle methicone et le stéaryle methicone, le cetyl dimethicone, le behenoxydimethicone;
- les dimethiconol ester de formule :

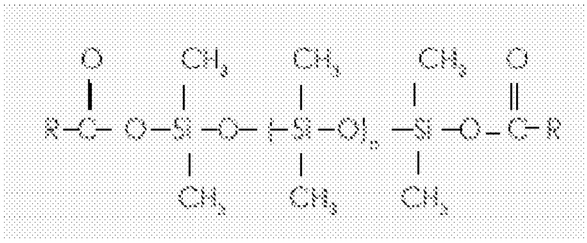

dans laquelle R est un radical alkyle ayant 6 à 32 atomes de carbone, tel que le dimethiconol behenate,

- les alkylamidoamines ayant notamment 6 à 60 atomes de carbone, notamment 12 à 50, telles que la behenamidopropyldimethylamine.
- les copolymères comprenant au moins une partie polyorganosiloxane et des groupements fluorés, et notamment les silicones fluorées ou fluorosilicones qui peuvent être représentées par la formule :

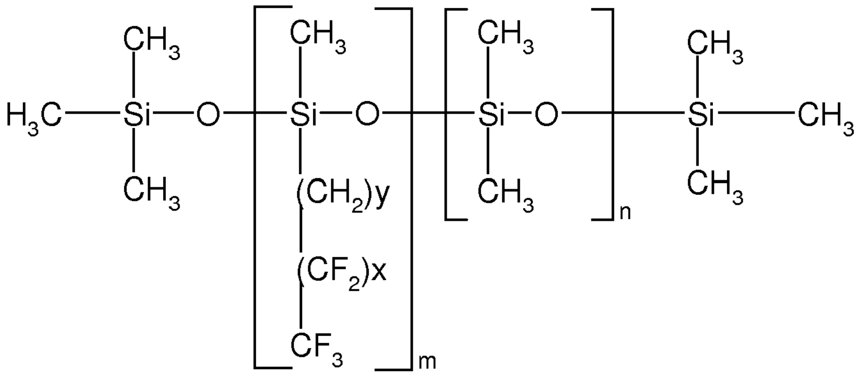

dans laquelle x est un entier compris entre 3 et 12, de préférence 5 et 10, notamment x=8; y est un entier compris entre 2 et 6, de préférence 2 ou 3; et m et n sont tels que le poids moléculaire du composé est compris entre 5000 et 15000;

- les copolymères blocs greffés ou séquencés comprenant au moins un bloc de type polyorganosiloxane et au moins un bloc polyéther, et notamment ceux pour lesquels le bloc polyorganopolysiloxane est un polydiméthylsiloxane ou un polyalkyl($C_2$-$C_{18}$) méthyl siloxane; le bloc polyéther est un polyalkylène en $C_2$-$C_{18}$, en particulier polyoxyéthylène et/ou polyoxypropylène.
- les diméthicones copolyol ou encore les alkyl ($C_2$-$C_{18}$) méthicones copolyol, éventuellement réticulés, tels que le lauryl méthicone copolyol; le lauryl diméthicone copolyol crosspolymer, le cétyl diméthicone copolyol et

le diméthicone copolyol PPG-3-oléyl éther;
- les copolymères de type "dibloc" ou "tribloc" du type polystyrène/polyisoprène, polystyrène/polybutadiène, du type polystyrène/copoly(éthylène-propylène) ou encore du type polystyrène/copoly(éthylène-butylène);
- les copolymères bi- ou triséquencés poly(méthacrylate de méthyle)/polyisobutylène ou les copolymères greffés à squelette poly(méthacrylate de méthyle) et à greffons polyisobutylène.
- les copolymères blocs greffés ou séquences comprenant au moins un bloc résultant de la polymérisation d'au moins un monomère éthylénique et d'au moins un bloc d'un polyéther tel qu'un polyoxyalkylène en C2-C18, en particulier polyoxyéthylène et/ou polyoxypropylène, tels que les copolymères bi- ou triséquencés polyoxyéthylène/polybutadiène ou polyoxyéthylène/polyisobutylène.

**22.** Composition selon l'une des revendications précédentes, dans laquelle l'agent stabilisant est choisi parmi les silicones fluorées ou fluorosilicones de formule :

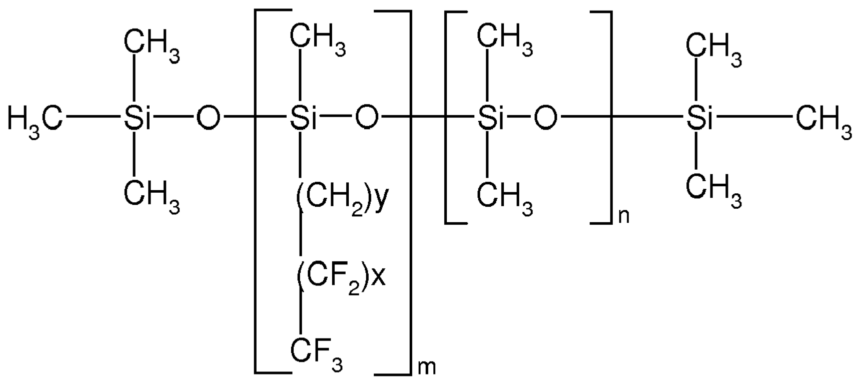

dans laquelle x=8, y = 2 ou 3, et m et n sont tels que le poids moléculaire du composé est compris entre 5000 et 15000.

**23.** Composition selon l'une des revendications précédentes, dans laquelle le polymère de la dispersion est présent à raison de 0,01 à 30% en poids de matière sèche, notamment de 0,1 à 20% en poids, voire de 0,1 à 10% en poids, encore mieux de 0,5 à 3% en poids, par rapport au poids total de la composition.

**24.** Composition selon l'une quelconque des revendications précédentes, **caractérisée par le fait que** l'agent tensioactif anionique est choisi parmi les sels (en particulier sels de métaux alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulfonates, paraffine-sulfonates; les alkylsulfosuccinates, les alkyléthersulfosuccinates, les alkylamidesulfosuccinates; les alkylsulfosuccinamates; les alkylsulfoacétates ; les alkylétherphosphates; les acylsarcosinates; les acyliséthionates et les N-acyltaurates, le radical alkyle ou acyle de tous ces différents composés comportant de préférence de 8 à 24 atomes de carbone, et le radical aryle désignant de préférence un groupement phényle ou benzyle, les sels d'acides gras tels que les sels des acides oléique, ricinoléique, palmitique, stéarique, les acides d'huile de coprah ou d'huile de coprah hydrogénée; les acyl-lactylates dont le radical acyle comporte 8 à 20 atomes de carbone; les acides d'alkyl D galactoside uroniques et leurs sels ainsi que les acides alkyl ($C_6$-$C_{24}$) éther carboxyliques polyoxyalkylénés, les acides alkyl ($C_6$-$C_{24}$)aryl éther carboxyliques polyoxyalkylénés, les acides alkyl($C_6$-$C_{24}$) amido éther carboxyliques polyoxyalkylénés et leurs sels, en particulier ceux comportant de 2 à 50 groupements oxyde d'éthylène, et leurs mélanges.

**25.** Composition selon l'une quelconque des revendications précédentes, **caractérisée par le fait que** la quantité de tensioactifs anioniques va de 3% à 25% en poids, en particulier de 5% à 25% en poids, rapportée au poids total de la composition cosmétique.

**26.** Composition selon l'une quelconque des revendications précédentes, **caractérisée par le fait que** l'agent tensioactif non ionique est choisi parmi les alcools, les alpha-diols, les alkylphénols ou les acides gras polyéthoxylés, polypropoxylés ou polyglycérolés, ayant une chaîne grasse comportant par exemple 8 à 22 atomes de carbone, le nombre de groupements oxyde d'éthylène ou oxyde de propylène pouvant aller notamment de 2 à 50 et le nombre de groupements glycérol pouvant aller notamment de 2 à 30, les copolymères d'oxyde d'éthylène et de propylène, les condensats d'oxyde d'éthylène et de propylène sur des alcools gras ; les amides gras polyéthoxylés ayant de

préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C10-C14) amines ou les oxydes de N-acylaminopropylmorpholine.

**27.** Composition selon l'une quelconque des revendications précédentes, **caractérisée par le fait que** la quantité de tensioactifs non ioniques va de 0.5% à 25% en poids, en particulier de 1 % à 20% en poids, et plus particulièrement de 2 à 10% en poids s, rapportée au poids total de la composition.

**28.** Composition selon l'une quelconque des revendications précédentes, **caractérisée par le fait que** l'agent tensioactif amphotère est choisi les dérivés d'amines secondaires ou tertiaires aliphatiques, dans lesquels le radical aliphatique est une chaîne linéaire ou ramifiée comportant 8 à 22 atomes de carbone et comprenant au moins un groupe anionique carboxylate, sulfonate, sulfate, phosphate ou phosphonate); les alkyl ($C_8$-$C_{20}$) bétaïnes, les sulfobétaïnes, les alkyl ($C_8$-$C_{20}$) amidoalkyl ($C_1$-$C_6$) bétaïnes telles que la cocoamidopropylbétaïne ou les alkyl ($C_8$-$C_{20}$) amidoalkyl ($C_1$-$C_6$) sulfobétaïnes et leurs mélanges.

**29.** Composition selon l'une quelconque des revendications précédentes, **caractérisée par le fait que** la quantité de tensioactifs amphotères va de 0.5% à 20% en poids, en particulier de 1% à 10% en poids, rapportée au poids total de la composition.

**30.** Composition selon l'une des revendications précédentes, **caractérisée par le fait que** la concentration totale des agents tensioactifs va de 0,1 % à 60% en poids, de préférence de 1% à 40% en poids, et encore plus préférentiellement de 5% à 30% en poids, par rapport au poids total de la composition.

**31.** Composition selon l'une quelconque des revendications précédentes, **caractérisée par le fait que** la composition comprend en outre au moins un polymère cationique.

**32.** Composition selon la revendication précédente, **caractérisée en ce que** le ou les polymères cationiques représentent de 0,001 % à 20 % en poids, de préférence de 0,01 % à 10% en poids par rapport au poids total de la composition finale.

**33.** Composition selon l'une quelconque des revendications précédentes, **caractérisée par le fait que** la composition comprend en outre au moins une silicone.

**34.** Composition selon la revendication précédente, **caractérisée en ce que** la ou les silicones représentent de 0,01 à 20 % en poids, de préférence de 0,1 à 5 % en poids par rapport au poids total de la composition.

**35.** Composition selon l'une quelconque des revendications précédentes, **caractérisée par le fait que** la composition comprend en outre au moins un additif choisi parmi les cires, les corps gras pâteux, les gommes et leurs mélanges, les huiles d'origine animale, végétale, minérale ou synthétique, les esters et les éthers de synthèse ; des alcools gras ayant de 12 à 26 atomes de carbone ; les huiles siliconées volatiles ou non, linéaires ou cycliques, liquides ou pâteux à température ambiante;les polymères différents des polymères de l'invention; les vitamines, les parfums, les agents nacrants, les épaississants, les gélifiants, les oligo-éléments, les adoucissants, les séquestrants, les parfums, les agents alcalinisants ou acidifiants, les conservateurs, les filtres solaires, les anti-oxydants, les agents anti-chutes des cheveux, les agents anti-pelliculaires, les agents anti-gras, les agents propulseurs, les céramides ; leurs mélanges.

**36.** Composition selon l'une des revendications précédentes, dans laquelle le milieu cosmétiquement acceptable comprend au moins un constituant choisi parmi l'eau ; les solvants organiques hydrophiles comme les alcools, notamment les monoalcools, linéaires ou ramifiés en C1-C6 et les polyols et les éthers de glycols

**37.** Composition selon l'une des revendications précédentes, se présentant sous la forme d'une composition capillaire, notamment pour le maintien de la coiffure ou la mise en forme des cheveux, par exemple sous forme de shampooings, de gels, de lotions de mise en plis, de lotions pour le brushing, de compositions de fixation et de coiffage telles que les laques ou spray ; d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

**38.** Procédé cosmétique de traitement des matières kératiniques telles que la peau du corps ou du visage, des ongles, des poils, des cheveux et/ou des cils, **caractérisé en ce qu'**il consiste à appliquer sur les matières kératiniques une composition cosmétique telle que définie dans l'une des revendications 1 à 37 et à faire suivre ou non l'application d'un rinçage après un éventuel temps de pause.

Office européen des brevets

# RAPPORT DE RECHERCHE EUROPEENNE

Numéro de la demande
EP 06 12 1666

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 5 916 548 A (HUTCHINS ET AL) 29 juin 1999 (1999-06-29) * colonne 2, ligne 1 - ligne 39; revendication 1; exemples 3,9-11,13 * * colonne 6, ligne 44 - colonne 9, ligne 28 * | 1-38 | INV. A61K8/04 A61K8/81 A61Q5/02 A61Q5/06 |
| X | EP 0 749 747 A (L'OREAL) 27 décembre 1996 (1996-12-27) * colonne 1, ligne 30 - colonne 5, ligne 52 * * colonne 8, ligne 14 - ligne 34; exemples 1-15 * | 1-38 | |
| A | US 2002/076390 A1 (KANTNER STEVEN S ET AL) 20 juin 2002 (2002-06-20) * page 1, alinéa 9 - alinéa 11; revendications * * page 2, alinéa 14 - alinéa 16 * * page 4, alinéa 38 - alinéa 40; exemple 1; tableau I * * page 6, alinéa 67 - alinéa 68 * * page 7, alinéa 82 - alinéa 84; exemples 18-20 * | 1-38 | DOMAINES TECHNIQUES RECHERCHES (IPC): A61K |
| A | EP 1 428 844 A (L'OREAL) 16 juin 2004 (2004-06-16) * page 2, alinéa 3 - page 3, alinéa 14; exemples 1-16 * * page 4, alinéa 23 - page 5, alinéa 25 * | 1-38 | |
| A | WO 98/31329 A (L'OREAL; MOUGIN, NATHALIE) 23 juillet 1998 (1998-07-23) * page 2, ligne 28 - page 4, ligne 10; revendications; exemples 1-9 * | 1-38 | |

Le présent rapport a été établi pour toutes les revendications

| Lieu de la recherche | Date d'achèvement de la recherche | Examinateur |
|---|---|---|
| Munich | 12 janvier 2007 | Loloiu, Camelia |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

6

## ANNEXE AU RAPPORT DE RECHERCHE EUROPEENNE RELATIF A LA DEMANDE DE BREVET EUROPEEN NO. EP 06 12 1666

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

12-01-2007

| Document brevet cité au rapport de recherche | Date de publication | Membre(s) de la famille de brevet(s) | Date de publication |
|---|---|---|---|
| US 5916548 A | 29-06-1999 | AUCUN | |
| EP 0749747 A | 27-12-1996 | AT 174502 T | 15-01-1999 |
| | | CA 2197496 A1 | 09-01-1997 |
| | | DE 69601147 D1 | 28-01-1999 |
| | | DE 69601147 T2 | 02-06-1999 |
| | | ES 2128149 T3 | 01-05-1999 |
| | | FR 2735689 A1 | 27-12-1996 |
| | | WO 9700663 A1 | 09-01-1997 |
| | | JP 2000044426 A | 15-02-2000 |
| | | JP 3225967 B2 | 05-11-2001 |
| | | JP 10501005 T | 27-01-1998 |
| | | US 5851517 A | 22-12-1998 |
| US 2002076390 A1 | 20-06-2002 | AUCUN | |
| EP 1428844 A | 16-06-2004 | BR 0305916 A | 31-08-2004 |
| | | CN 1513886 A | 21-07-2004 |
| | | JP 2004190034 A | 08-07-2004 |
| | | KR 20040051571 A | 18-06-2004 |
| | | MX PA03011445 A | 19-04-2005 |
| | | US 2004156812 A1 | 12-08-2004 |
| WO 9831329 A | 23-07-1998 | CA 2249478 A1 | 23-07-1998 |
| | | CN 1224343 A | 28-07-1999 |
| | | EP 0909157 A1 | 21-04-1999 |
| | | JP 2002501481 T | 15-01-2002 |
| | | US 6126929 A | 03-10-2000 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9115185 A
- WO 9115186 A
- WO 9707782 A
- EP 749747 A
- EP 0337354 A
- FR 2270846 A
- FR 2383660 A
- FR 2598611 A
- FR 2470596 A
- FR 2519863 A
- FR 2505348
- FR 2542997
- EP 080976 A
- FR 277143
- FR 2393573
- FR 1492597
- US 4131576 A
- US 3589578 A
- US 4031307 A
- FR 216225
- FR 2280361
- FR 2252840
- FR 2368508
- FR 1583363
- FR 280759
- FR 2190406
- FR 2320330
- FR 2270846
- FR 2316271
- FR 2336434
- FR 2413907
- US 2273780 A
- US 2375853 A
- US 2388614 A
- US 2454547 A
- US 3206462 A
- US 226102 A
- US 2271378 A
- US 3874870 A
- US 401432 A
- US 3929990 A
- US 3966904 A
- US 405193 A
- US 425617 A
- US 425627 A
- US 425653 A
- US 426945 A
- US 42720 A
- EP 122324 A
- FR 8516334 A
- US 4957732 A
- EP 186507 A
- EP 342834 A


### Littérature non-brevet citée dans la description


- Polymer Handbook. John Wiley, 1989
- Solubility parameter values. **GRULKE.** Polymer Handbook. 519-559
- **HANSEN.** The three dimensionnal solubility parameters. *J. Paint Technol.,* 1967, vol. 39, 105
- **WALTER NOLL.** Chemistry and Technology of Silicones. Academie Press, 1968
- Volatile Silicone fluids for cosmetics. **TODD ; BYERS.** Cosmetics and toiletries. vol. 91, 27-32